# EUROPEAN PATENT SPECIFICATION

(11) **EP 3 391 872 B1**
(45) Date of publication and mention of the grant of the patent: **14.09.2022**
(21) Application number: 16875711.0
(22) Date of filing: 15.12.2016
(51) Int. Cl.: A61K 8/33, A61K 8/06, A61K 8/37, A61K 8/44, A61K 8/60, A61K 8/64, A61Q 19/00, B01J 13/00, A61K 8/39, A61K 8/55, A61K 8/86

(54) **OIL-IN-WATER TYPE EMULSION COMPOSITION AND METHOD FOR PRODUCING SAME**
ZUSAMMENSETZUNG VOM ÖL-IN-WASSER-EMULSIONSTYP UND VERFAHREN ZUR HERSTELLUNG DAVON
COMPOSITION DE TYPE ÉMULSION HUILE DANS L'EAU ET SON PROCÉDÉ DE PRODUCTION

(30) Priority: 18.12.2015 JP 2015247937; 18.12.2015 JP 2015247782; 21.12.2015 JP 2015248846; 21.12.2015 JP 2015248855; 28.10.2016 JP 2016211238
(43) Date of publication of application: 24.10.2018
(73) Proprietor: Pola Chemical Industries, Inc., Shizuoka 437-8765 (JP)
(72) Inventor: NIOH, Atsushi, Yokohama-shi Kanagawa 244-0812 (JP); MATSUO, Kazuki, Yokohama-shi Kanagawa 244-0812 (JP); FUJIYAMA, Ippei, Yokohama-shi Kanagawa 244-0812 (JP); GOTO, Haruka, Yokohama-shi Kanagawa 244-0812 (JP)
(74) Representative: Jones, Nicholas Andrew
(86) International application number: PCT/JP2016/087336
(87) International publication number: WO 2017/104734

(56) References cited:
- JP-A- H 054 912
- JP-A- H 054 912
- JP-A- S5 946 123
- JP-A- H04 253 903
- JP-A- 2006 117 643
- JP-A- 2009 234 971
- JP-A- 2009 234 971
- JP-A- 2009 298 748
- JP-A- 2011 168 548
- JP-A- 2014 073 991
- JP-A- 2014 073 991
- JP-A- 2015 134 741
- JP-B2- H0 251 665

## Description

### TECHNICAL FIELD

The present invention relates to an oil-in-water type emulsion composition suitably for skin external preparations, and a method of producing the same.

### BACKGROUND ART

Dosage forms of skin external preparations such as milky lotions and creams include emulsions, and an oil-in-water type emulsions are particularly preferred since they yield a fresh feeling of use.

An emulsion is an unstable dispersion system in a high energy state, and even the same formulation yields different states depending on the preparation method. Therefore, a variety of emulsification methods have been proposed and, generally speaking, surface chemical techniques utilizing the interfacial tension-reducing ability of surfactants and mechanical techniques utilizing a mechanical energy of an emulsification apparatus have been employed.

Usually, in emulsification carried out by a surface chemical technique, the HLB (Hydrophilic-Lipophilic Balance) value of a surfactant is used as an index of emulsification, and the adjustment thereof more or less depends on the experience.

Further, emulsification methods using superaggregates of molecules have been developed. For instance, D-phase emulsification and liquid-crystal emulsification generate fine emulsified particles by dispersing and retaining a dispersion phase in a liquid crystal or D-phase formed by surfactants, and these methods are advantageous in that the adjustment of the HLB value is not much required (Non-patent Documents 1 and 2).

### PRIOR ART REFERENCES

### NON-PATENT DOCUMENTS

[Non-patent Document 1] Masayuki Endo, Hiromichi Sagitani, Journal of Japan Oil Chemists' Society, vol. 40, no. 2, 1991: 133-139
[Non-patent Document 2] Toshiyuki Suzuki, Masanobu Kai, Atsuo Ishida, Journal of Japan Oil Chemists' Society, vol. 34, no. 11, 1985: 938-945

### SUMMARY OF THE INVENTION

### PROBLEMS TO BE SOLVED BY THE INVENTION

However, even with the use of such a conventional emulsification method, it is difficult to incorporate various kinds of oil agents in the internal phase of an oil-in-water type emulsion, and the formulation design has limited freedom. Particularly, among oil agents, it is not easy to emulsify a large amount of a low-molecular-weight polar oil that yields a refreshing feel. In addition, since high polarity of an oil generally makes a surfactant more easily dissolve in the oil, the surfactant must be incorporated in a large amount in order to improve the emulsion stability, and this is not preferable since it causes filminess when the emulsion is applied to the skin.

Moreover, an emulsification method which generates a finer emulsion and further improves the temporal and temperature stability is desired.

In view of the above-described circumstances, an object of the present invention is to provide a useful emulsification framework capable of emulsifying various kinds of oil agents. Another object of the present invention is to provide, using the emulsification framework, an oil-in-water type emulsion composition which has excellent temporal and temperature stability and exerts a good feeling of use, and a method of producing the same.

### MEANS FOR SOLVING THE PROBLEMS

The present inventors intensively studied to solve the above-described problems and consequently discovered that the problems can be solved by carrying out emulsification through a specific intermediate phase formed by mixing a surfactant capable of forming an L_{α} phase, a surfactant capable of forming an H₁ phase, and water, thereby completing the present invention.

That is, the present invention relates to a method of producing an oil-in-water type emulsion composition, the method comprising: the step of forming an intermediate phase by mixing a first surfactant, a second surfactant, and water; the step of adding an oil phase component to the intermediate phase; and the step of further adding an aqueous phase component, wherein the first surfactant forms an L_{α} phase in at least one aqueous solution having a concentration of 40 to 80% by mass, and the second surfactant forms an H₁ phase in at least one aqueous solution having a concentration of 40 to 80% by mass, wherein the total concentration of said first and second surfactants in said intermediate phase is 20 to 85% by mass.

In this embodiment, preferably, the intermediate phase has an interfacial tension of 0.01 to 8 mN/m in terms of relative value with respect to polyperfluoromethylisopropyl ether.

In this embodiment, preferably, a combination of the first surfactant and the second surfactant is a combination in which the viscosity of a mixed aqueous solution of the first and second surfactants is less than half of the sum of the viscosity values of individual aqueous solutions of the first and second surfactants at the same concentration as the total concentration of the surfactants in the mixed aqueous solution.

In this embodiment, preferably, the concentration of the first surfactant is 0.1 to 5% by mass with respect to the whole composition.

In this embodiment, preferably, the concentration of the second surfactant is 0.1 to 5% by mass with respect to the whole composition.

In this embodiment, preferably, the total concentration of the first and second surfactants is 0.2 to 8% by mass with respect to the whole composition.

In this embodiment, preferably, the mass ratio of the first surfactant and the second surfactant is 2:8 to 8:2.

In this embodiment, the first surfactant comprises preferably at least one selected from surfactin and salts thereof, mannosylerythritol lipids, polyglycerol fatty acid esters, sophorolipids, polyoxyethylene glyceryl fatty acids, polyethylene glycol difatty acids, and phospholipids, more preferably a polyglycerol fatty acid ester or a phospholipid.

In this embodiment, the second surfactant comprises preferably at least one selected from alkyl glycosides represented by the following Formula (1), acylamino acid lysine and/or salts thereof, sucrose fatty acid esters, polyglycerol alkyl ethers, fatty acid glycosides, and monoalkyl phosphates and salts thereof, more preferably an alkyl glycoside, or a monoalkyl phosphate and a salt thereof: 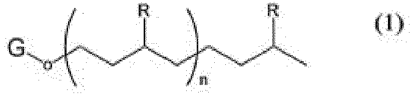 wherein G represents a sugar residue; Rs independently represent a hydrogen atom or an alkyl group having 1 to 3 carbon atoms; and n represents an integer of 1 to 4.

In this embodiment, preferably, the first surfactant and/or the second surfactant comprise no ethylene oxide (EO) group.

In this embodiment, preferably, the content of the oil phase component is 3 to 75% by mass with respect to the whole composition.

In this embodiment, preferably, the method comprises the step of incorporating a polyhydric alcohol in an amount of 1 to 30% by mass with respect to the whole composition.

In this embodiment, the oil-in-water type emulsion composition is preferably a skin external preparation, more preferably a cosmetic. Further, the skin external preparation is preferably one used for maturation of skin horny layer.

A second embodiment of the present invention is an oil-in-water type emulsion composition comprising: a first surfactant; and a second surfactant, wherein the first surfactant forms an L_{α} phase in at least one aqueous solution having a concentration of 40 to 80% by mass, and the second surfactant forms an H₁ phase in at least one aqueous solution having a concentration of 40 to 80% by mass.

A third embodiment of the present invention is an oil-in-water type emulsion composition comprising: at least one alkyl glycoside represented by the following Formula (1); and at least one selected from surfactin and salts thereof, mannosylerythritol lipids, polyglycerol fatty acid esters, sophorolipids, polyoxyethylene glyceryl fatty acids, polyethylene glycol difatty acids, and phospholipids: 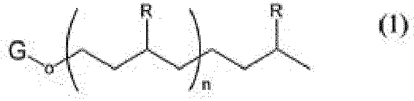 (wherein, G represents a sugar residue; Rs independently represent a hydrogen atom or an alkyl group having 1 to 3 carbon atoms; and n represents an integer of 1 to 4).

A fourth embodiment of the present invention is an oil-in-water type emulsion composition comprising: arginine dodecylphosphate; and at least one selected from surfactin and salts thereof, mannosylerythritol lipids, polyglycerol fatty acid esters, sophorolipids, polyoxyethylene glyceryl fatty acids, polyethylene glycol difatty acids, and phospholipids.

A fifth embodiment of the present invention is an oil-in-water type emulsion composition comprising: acylamino acid lysine and/or a salt thereof; and at least one selected from surfactin and salts thereof, mannosylerythritol lipids, and polyglycerol fatty acid esters.

A sixth embodiment of the present invention is an oil-in-water type emulsion composition comprising: a sucrose fatty acid ester; and at least one selected from surfactin and salts thereof, mannosylerythritol lipids, and polyglycerol fatty acid esters.

In this embodiment, preferably, the sucrose fatty acid ester is sucrose laurate.

A seventh embodiment of the present invention is an oil-in-water type emulsion composition comprising: a polyglycerol alkyl ether; and at least one selected from surfactin and salts thereof, mannosylerythritol lipids, and polyglycerol fatty acid esters.

In this embodiment, preferably, the polyglycerol alkyl ether is a polyglycerol monolauryl ether.

In the third to seventh embodiments of the present invention, preferably, the polyglycerol fatty acid esters are polyglyceryl monolaurates having a glycerol polymerization degree of 4 to 6.

In the second to seventh embodiments of the present invention, the oil-in-water type emulsion composition is preferably a skin external preparation, more preferably a cosmetic. Further, the skin external preparation is preferably one used for maturation of skin horny layer.

### EFFECTS OF THE INVENTION

By the present invention, an intermediate phase which can be a useful emulsification framework capable of emulsifying various kinds of oil agents is provided. Particularly, since the intermediate phase according to the present invention can contain a large amount of a low-molecular-weight polar oil in its internal phase, an oil-in-water type emulsion composition produced through this intermediate phase yields a refreshing feeling. Further, through the intermediate phase, emulsification can be carried out with a small amount of surfactants, so that the filminess is suppressed and a good feeling of use can be obtained when the resulting oil-in-water type emulsion composition is applied to the skin. Moreover, according to the present invention, since a fine emulsion can be formed, the resulting oil-in-water type emulsion composition has excellent temporal and temperature stability.

### BRIEF DESCRIPTION OF THE DRAWINGS

[FIG. 1] FIG. 1 shows (a) an optical micrograph of tape-stripped horny layer cells of an untreated human epidermis model, and (b) an optical micrograph of the horny layer cells (drawing-substituting photographs).
[FIG. 2] FIG. 2 shows (a) an optical micrograph of tape-stripped horny layer cells of a human epidermis model to which the oil-in-water type emulsion composition of Reference Example 1 was applied, and (b) an optical micrograph of the horny layer cells (drawing-substituting photographs).
[FIG. 3] FIG. 3 shows (a) an optical micrograph of tape-stripped horny layer cells of a human epidermis model to which the oil-in-water type emulsion composition of Example 53 was applied, and (b) an optical micrograph of the horny layer cells (drawing-substituting photographs).
[FIG. 4] FIG. 4 is a graph showing the temporal change in TEWL of the skin to which the oil-in-water type emulsion composition of Example 54 or Reference Example 2 was applied.
[FIG. 5] FIG. 5 is a graph showing the elasticity of the skin to which the oil-in-water type emulsion composition of Example 54 or Reference Example 2 was applied.

### MODE FOR CARRYING OUT THE INVENTION

### <Production Method of Present Invention>

The production method according to the first embodiment of the present invention comprises the step of forming an intermediate phase by mixing a surfactant capable of forming an L_{α} phase, a surfactant capable of forming an H₁ phase, and water. The intermediate phase has the below-described specific physical properties and can be a useful emulsification framework that is capable of emulsifying various kinds of oil agents and enables to carry out emulsification even by manual stirring without the use of a sophisticated emulsification apparatus. The production method is innovative in that it can yield the above-described useful emulsification framework by selecting appropriate surfactants based on a simple standard, even without performing complicated HLB value adjustment.

In addition, the production method of this embodiment sequentially comprises, after the step of forming an intermediate phase; the step of adding an oil phase component to a mixture in which the intermediate phase has been formed; and the step of further adding an aqueous phase component. These steps can be carried out at any temperature; however, from the standpoint of uniformly mixing the components, it is preferred to carry out these steps by mixing the components with heating at 40 to 80°C. Further, these steps are preferably carried out with stirring, and the stirring may be manual stirring or mechanical stirring.

In this embodiment, the step of forming an intermediate phase is carried out by mixing a first surfactant, a second surfactant, and water. In this case, the mixing order is not particularly restricted, and an aqueous solution containing the first surfactant and an aqueous solution containing the second surfactant may be mixed; the second surfactant may be added to an aqueous solution containing the first surfactant; the first surfactant may be added to an aqueous solution containing the second surfactant; or the first and second surfactants may be simultaneously added to water.

In this embodiment, the interfacial tension of the intermediate phase is preferably small and, specifically, it is 0.01 to 8 mN/m, preferably 0.1 to 8 mN/m, more preferably 0.1 to 5 mN/m, in terms of relative value with respect to polyperfluoromethylisopropyl ether (FOMBLIN HC/04, manufactured by Nikko Chemicals Co., Ltd.). By forming such a low-interfacial-tension intermediate phase, the surfactant molecules are efficiently oriented at an oil-water interface and the oil phase component is efficiently emulsified in the subsequent steps, so that a fine emulsion can be generated.

More specifically, for example, when the below-described polyglyceryl-5 oleate is used as the first surfactant and the below-described Mal₂Far is used as the second surfactant, the interfacial tension of a mixture containing the intermediate phase is preferably 0.01 to 5 mN/m in terms of relative value with respect to polyperfluoromethylisopropyl ether.

It is noted there that the interfacial tension is a value measured by a spinning-drop interfacial tension measurement method (apparatus: SITE100MK2, manufactured by KRÜSS GmbH; measurement temperature: 20°C).

For the formation of the intermediate phase according to this embodiment, two surfactants having different properties are used in combination. That is, a surfactant which forms an L_{α} phase at any one temperature of 15 to 80°C in at least one aqueous solution having a concentration of 40 to 80% by mass is used as the first surfactant, and a surfactant which forms an H₁ phase at any one temperature of 15 to 80°C in at least one aqueous solution having a concentration of 40 to 80% by mass is used as the second surfactant. However, the points that the first surfactant may form an L_{α} phase outside the above-described concentration range and that the second surfactant may form an H₁ phase outside the above-described concentration range are not excluded. Further, the points that the first surfactant does not form an L_{α} phase at a certain concentration within the above-described concentration range and that the second surfactant does not form an H₁ phase at a certain concentration within the above-described concentration range are also not excluded.

In the present specification, the term "L_{α} phase" refers to a lamellar liquid crystal phase, and the term "H₁ phase" refers to a hexagonal liquid crystal phase. The formation of these liquid crystal phases can be confirmed by a conventional method, such as X-ray diffraction analysis or polarizing microscopy.

In this embodiment, the combination of the first surfactant and the second surfactant is preferably one which has a lower viscosity as a mixed aqueous solution of the two surfactants than those of individual aqueous solutions of each surfactant. More specifically, the combination is one in which the viscosity of a mixed aqueous solution of the first and second surfactants is preferably less than 1/2, more preferably less than 1/5, still more preferably less than 1/10, of the sum of the viscosity values of individual aqueous solutions of the first and second surfactants at the same concentration as the total concentration of the surfactants in the mixed aqueous solution. It is noted here that, as long as the combination of the first and second surfactants is one which has a lower viscosity as a mixed aqueous solution of the two surfactants than those of individual aqueous solutions of each surfactant at an arbitrary mixing ratio or total concentration, such a combination is not excluded as unpreferable even if there is a mixing ratio or total concentration at which a reduction in viscosity does not occur.

By using two surfactants in a combination having such a property, since the surfactant molecules are efficiently oriented at an oil-water interface when the oil phase component is added to the intermediate phase and the interfacial tension is thereby reduced, the oil phase component is efficiently emulsified, so that a fine emulsion can be generated.

It is noted there that the viscosity is a value measured by a viscoelasticity measurement method using a rheometer (apparatus: AR-G2, manufactured by TA Instruments Japan Inc.; measurement mode: stress sweep, measurement temperature: 20°C, vibrational stress: 1 to 600 Pa, geometry: 40-mm cone, GAP value: 1,000).

In the mixture forming the intermediate phase according to this embodiment, the total concentration of the first and second surfactants is preferably 20 to 85% by mass, more preferably 35 to 75% by mass, still more preferably 40 to 65% by mass.

When the total concentration is less than 20% by mass or higher than 85% by mass, the intermediate phase may not be properly formed and/or the amount of an oil agent to be added may be restricted, making the subsequent emulsification difficult.

In this embodiment, the concentration of the first surfactant is preferably 0.1 to 5% by mass, more preferably 0.1 to 3% by mass, still more preferably 0.3 to 2% by mass, with respect to the whole composition. Further, in this embodiment, the concentration of the second surfactant is preferably 0.1 to 5% by mass, more preferably 0.1 to 3% by mass, still more preferably 0.3 to 2% by mass, with respect to the whole composition.

The total concentration of the first and the second surfactants is preferably 0.2 to 8% by mass, more preferably 0.2 to 6% by mass, still more preferably 0.6 to 4% by mass, with respect to the whole composition. When the total concentration is less than 0.2% by mass, the emulsion stability may be impaired, whereas when the total concentration is higher than 8% by mass, application of the resulting composition to the skin may cause filminess.

Moreover, in this embodiment, the mass ratio at which the first surfactant and the second surfactant are used is preferably 2:8 to 8:2, more preferably 4:6 to 8:2. By using a combination of two surfactants having different properties at such a mass ratio, a specific intermediate phase is easily formed, and the subsequently emulsification can thus be easily carried out.

### <First Surfactant Capable of Forming L_{α} Phase>

In the present invention, it is preferred that the first surfactant comprises at least one selected from surfactin and salts thereof, mannosylerythritol lipids, polyglycerol fatty acid esters, sophorolipids, polyoxyethylene glyceryl fatty acids, polyethylene glycol difatty acids, and phospholipids.

Surfactin that can be used as the first surfactant is a so-called biosurfactant and refers to 2-deamino-3-(10-methylundecyl)-cyclo[L-Ser^{∗}-L-Glu-L-Leu-D-Leu-L-Val-L-Asp-D-L eu-L-Leu-], which is a cyclic peptide. As a salt of surfactin, a sodium salt can be preferably used in the present invention.

The mannosylerythritol lipids (MELs) that can be used as the first surfactant are also biosurfactants. Any of MEL-A, B, C and D can be used in the present invention, and MEL-B is particularly preferred.

The polyglycerol fatty acid esters that can be used as the first surfactant are esters of a branched or linear, saturated or unsaturated fatty acid and glycerol, and the fatty acid has preferably 8 to 20 carbon atoms, more preferably 12 to 18 carbon atoms. The glycerol polymerization degree is preferably 2 to 10, more preferably 4 to 10, still more preferably 4 to 6. Further, the fatty acid addition number is not particularly restricted, and it may be a mono-, di-, tri- or higher ester; however, a monoester is preferred. The HLB value of the first surfactant is also not particularly restricted; however, it is preferably 3 to 18. In the present specification, the HLB value refers to a value calculated by the Griffin equation.

Further, as an ester which is likely to form an L_{α} phase, that is, an ester which is easily arranged on a flat plate because of the polarity of its molecules, the critical packing parameter (CPP) which represents the geometrical ratio of the hydrophilic moiety and the hydrophobic moiety of an amphiphile is preferably 1/2 to 1.

Preferred examples of such polyglycerol fatty acid esters include polyglyceryl monooleate, polyglyceryl monolaurate, polyglyceryl monopalmitate, polyglyceryl monoisostearate, and polyglyceryl dilaurate. More preferred examples include polyglyceryl monooleate (e.g., polyglyceryl-5 monooleate), polyglyceryl monolaurate (e.g., polyglyceryl-4 monolaurate, polyglyceryl-6 monolaurate, and polyglyceryl-10 monolaurate) and polyglyceryl monopalmitate (e.g., polyglyceryl-6 monopalmitate), which have a glycerol polymerization degree of 4 to 10. Still more preferred examples include polyglyceryl monolaurate having a glycerol polymerization degree of 4 to 6 (e.g., polyglyceryl-4 monolaurate and polyglyceryl-6 monolaurate).

Sophorolipids are so-called biosurfactants and refer to glycolipids consisting of a hydroxyl fatty acid and sophorose or sophorose in which some of hydroxyl groups are acetylated. Generally speaking, sophorolipids exist in the form of a mixture of a lactone ring-containing molecule (lactone-type) and a ring-opened molecule thereof (acid-type). In the present invention, any of such sophorolipids may be used, and the mixing ratio thereof is not particularly restricted.

The fatty acids constituting the polyoxyethylene glyceryl fatty acids that can be used as the first surfactant are preferably branched or linear, saturated or unsaturated fatty acids having 8 to 20 carbon atoms, more preferably branched fatty acids having 18 carbon atoms. The average number of moles of added oxyethylene in the polyoxyethylene group is preferably 5 to 30, more preferably 10 to 25. Further, a fatty acid residue may be added to only one, or two or three of the three hydroxyl groups of the polyoxyethylene-added glyceryl group. A structure in which three fatty acid residues are added is preferred; however, a structure which contains two or more selected from a monoadduct, a diadduct and a triadduct is also preferred.

Examples of commercially available products include "EMALEX GWIS-320" (manufactured by Nihon Emulsion Co., Ltd.), which is polyoxyethylene (20) glyceryl triisostearate.

The fatty acids constituting the polyethylene glycol difatty acids that can be used as the first surfactant are preferably branched or linear, saturated or unsaturated fatty acids having 8 to 20 carbon atoms, more preferably branched fatty acids having 18 carbon atoms. Further, the average number of moles of added ethylene oxide is preferably 3 to 60, more preferably 5 to 30, still more preferably 10 to 15.

Such polyethylene glycol difatty acids are commercially available, and examples thereof include polyethylene glycol dilaurate (6EO, HLB = 7, EMALEX 300di-L), polyethylene glycol dilaurate (8EO, HLB = 8, EMALEX DEG-di-L), polyethylene glycol dilaurate (12EO, HLB = 10, EMALEX600di-L), polyethylene glycol distearate (3EO, HLB = 3, EMALEX TEG-di-S), polyethylene glycol distearate (12EO, HLB = 8, EMALEX 600di-SD), polyethylene glycol diisostearate (12EO, HLB = 8, EMALEX 600di-ISEX) and polyethylene glycol dioleate (12EO, HLB = 8, EMALEX 600di-OD), which are manufactured by Nihon Emulsion Co., Ltd. These products may be used individually, or in combination of two or more thereof.

Examples of phospholipids that can be used as the first surfactant include glycerophospholipids and sphingophospholipids.

Glycerophospholipids are substances having a glycerophosphate skeleton and comprise a fatty acid ester, a long-chain alkyl ether, vinyl ether or the like as a lipophilic group. Specific examples thereof include phosphatidyl choline (lecithin), phosphatidylethanolamine, phosphatidyl serine, phosphadiyl inositol, phosphatidyl inositol polyphosphate, phosphatidyl glycerol, diphosphatidyl glycerol (cardiolipin), phosphatidic acid, lysophosphatidyl choline, lysophosphatidylethanolamine, lysophosphatidyl serine, lysophosphatidyl inositol, lysophosphatidyl glycerol, and lysophosphatidic acid.

Sphingophospholipids comprise a long-chain base or long-chain fatty acid, such as sphingosine or phytosphingosine, and phosphoric acid or phosphonic acid. Specific examples thereof include ceramide 1-phosphate derivatives (e.g., sphingomyelin) and ceramide 1-phosphonate derivatives (e.g., ceramide aminoethylphosphonate).

In the present invention, the phospholipids may be natural products that are extracted and refined from animals and plants, phospholipids that are chemically synthesized, or phospholipids that are subjected to a processing such as hydrogenation or hydroxylation. Preferred examples of such phospholipids include hydrogenated lecithins. As the natural products, lecithins that are extracted and refined from soybean, egg yolk or the like are preferred because of their availability as commercial products.

In a more preferred embodiment of the present invention, a surfactant having no ethylene oxide (EO) group is used as the first surfactant.

### <Second Surfactant Capable of Forming H₁ Phase>

In the present invention, the second surfactant comprises preferably at least one selected from alkyl glycosides represented by the following Formula (1), acylamino acid lysine and/or salts thereof, sucrose fatty acid esters, polyglycerol alkyl ethers, fatty acid glycosides, and monoalkyl phosphates and salts thereof, more preferably an alkyl glycoside represented by the following Formula (1): 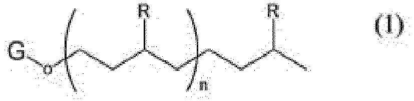 (wherein, G represents a sugar residue; Rs independently represent a hydrogen atom or an alkyl group having 1 to 3 carbon atoms; and n represents an integer of 1 to 4).

As the acylamino acid lysine and/or salts thereof that can be used as the second surfactants, for example, lysine diacyl glutamate whose acyl groups are derived from a fatty acid is preferred, and lysine dilauroyl glutamate, lysine dimyristoyl glutamate, lysine distearoyl glutamate, lysine dilinoleoyl glutamate and the like are more preferred.

Examples of salts thereof include alkali metal salts, such as sodium salts and potassium salts; organic amine salts, such as triethanolamine salts; and basic amino acid salts, such as arginine salts.

These can be obtained by a synthesis reaction between lysine hydrochloride and an acylamino acid anhydride, or a commercially available product can be used.

Preferred examples of the sucrose fatty acid esters that can be used as the second surfactant include sucrose laurate, sucrose myristate, sucrose palmitate, sucrose stearate and sucrose oleate, among which sucrose laurate is more preferred. As these sucrose fatty acid esters, commercially available products can be used as well.

Preferred examples of the polyglycerol alkyl ethers that can be used as the second surfactant include polyglycerol monolauryl ether, polyglycerol monomyristyl ether, polyglycerol monopalmityl ether, polyglycerol monostearyl ether, polyglycerol monoisostearyl ether and polyglycerol monooleyl ether, among which polyglycerol monolauryl ether is more preferred. The glycerol polymerization degree in these polyglycerol alkyl ethers is preferably 2 to 10, more preferably 3 to 6, still more preferably 4. As these polyglycerol alkyl ethers, commercially available products can be used as well.

The alkyl glycosides represented by the Formula (1) that can be used as the second surfactant have a structure in which a sugar residue G and an aliphatic alcohol are condensed.

The sugar residue G constitutes a hydrophilic moiety in the alkyl glycosides represented by the Formula (1) and is a residue of a monosaccharide or polysaccharide.

Preferred examples of the monosaccharide include glucose, galactose, xylose, mannose, lyxose, arabinose, and fructose. The polysaccharide is preferably a di- to hepta-saccharide, and examples thereof include maltose, xylobiose, isomaltose, cellobiose, lactose, gentiobiose, maltotriose, isomaltotriose, cellotriose, maltotetraose, maltopentaose, maltohexaose, maltoheptaose, isomaltotetraose, isomaltopentaose, isomaltohexaose, and isomaltoheptaose. Among these saccharides, polysaccharides are more preferred, glucose, galactose, maltose and maltotriose are still more preferred, and maltose and maltotriose are particularly preferred. The sugar residue may be in the *α*-form or the *β*-form.

In the Formula (1), the sugar residue G is bound (condensed) with the aliphatic alcohol, preferably via the hydroxyl group at the 1-position of the terminal sugar.

In the Formula (1), the aliphatic chain constitutes a hydrophobic moiety in the alkyl glycosides represented by the Formula (1).

The aliphatic chain portion does not have to contain a branched; however, it preferably has a branched chain. That is, in the Formula (1), Rs independently represent a hydrogen atom or an alkyl group having 1 to 3 carbon atoms, preferably an alkyl group having 1 to 3 carbon atoms, more preferably a methyl group.

As the alkyl glycosides represented by the Formula (1), compounds represented by the following Formula (2) are more preferred.

In the Formula (2), N represents an integer of 1 to 7; Rs independently represent a hydrogen atom or an alkyl group having 1 to 3 carbon atoms, preferably a methyl group; and n represents an integer of 1 to 4, preferably 2 or 3.

Specific examples of alkyl glycosides represented by the Formula (2) include a glycoside of maltose and hexahydrofarnesol (Mal₂Far), a glycoside of maltotriose and hexahydrofarnesol (Mal₃Far), and a glycoside of maltotriose and dihydrophytol (Mal₃Phyt).

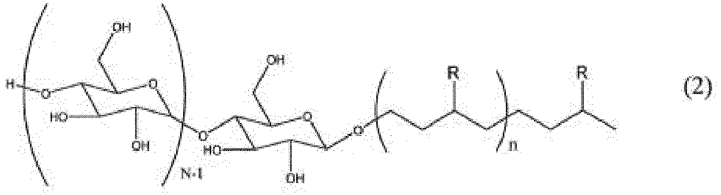

The alkyl glycosides represented by the Formula (1) can be obtained by a variety of well-known methods, such as glycosylation using a brominated sugar, glycosylation using a fluorinated sugar, glycosylation using trichloroacetimidate and glycosylation using an acetylated sugar, and more specific examples of the methods include the synthesis steps described in Japanese Patent No. 3882067, Japanese Patent No. 4817435, Japanese Patent No. 5207420, Japanese Laid-open Patent Application (Kokai) No. 2013-129660, Japanese Laid-open Patent Application (Kokai) No. 2012-17318 and the like.

In recent years, taking into consideration the safety and the stress to the environment, so-called natural surfactants which are renewable and believed to impose less environmental stress tend to be more preferred over conventional synthetic surfactants derived from petroleum materials. Therefore, in the present invention, in order to comply with such demand, it is particularly preferred to use an alkyl glycoside represented by the Formula (1) as the second surfactant; however, the second surfactant is not restricted thereto.

As the fatty acid glycosides that can be used as the second surfactant, esters formed by a branched or linear, saturated or unsaturated fatty acid having 10 to 16 carbon atoms and a residue of a monosaccharide or polysaccharide are preferred, and the sugar residue is preferably bound (ester-condensed) with the fatty acid via the hydroxyl group at the 1-position of the terminal sugar. As the fatty acid glycosides, ones having an HLB value of 12 to 18 are more preferred.

Preferred examples of the monosaccharide include glucose, galactose, xylose, mannose, lyxose, arabinose, and fructose. The polysaccharide is preferably a di- to hepta-saccharide, and examples thereof include maltose, xylobiose, isomaltose, cellobiose, lactose, gentiobiose, maltotriose, isomaltotriose, cellotriose, maltotetraose, maltopentaose, maltohexaose, maltoheptaose, isomaltotetraose, isomaltopentaose, isomaltohexaose, and isomaltoheptaose. Among these saccharides, glucose, galactose, maltose and maltotriose are more preferred, and maltose and maltotriose are still more preferred. The sugar residue may be in the *α*-form or the *β*-form.

Preferred examples of the fatty acid glycosides include lauroyl maltoside, nonanoyl maltoside, undecanoyl maltoside, tridecanoyl maltoside, and tetradecanoyl maltoside.

As the monoalkyl phosphates and salts thereof that can be used as the second surfactant, esters formed by a branched or linear, saturated or unsaturated aliphatic chain having preferably 8 to 20 carbon atoms, more preferably 12 to 18 carbon atoms, still more preferably 12 carbon atoms, and phosphoric acid are preferred, and examples of the salts thereof include sodium salts, potassium salts, ammonium salts, monoethanol ammonium salts, diethanol ammonium salts, triethanol ammonium salts and arginine salts, among which arginine salts are preferred. Further, ethylene oxide may be added thereto; however, it is preferred that no ethylene oxide is added thereto.

In a more preferred embodiment of the present invention, a surfactant having no ethylene oxide (EO) group is used as the second surfactant.

### <Oil Phase Component>

In the present invention, the oil phase component constitutes an internal phase of the oil-in-water type emulsion composition.

The oil phase component is usually composed of an oily component, and the oily component is not restricted to a so-called oil agent and refers to a component (excluding a surfactant) which shows phase separation from water when it is suspended in water at 25 to 65°C and left to stand for 1 hour.

Examples of an oily component that can be incorporated into the oil-in-water type emulsion composition of the present invention include oil agents, such as polar oils, natural oils, hydrocarbon oils, non-polar silicone oils and polar silicone oils; UV protection agents, such as UV absorbers and UV scattering agents; and oil-soluble vitamins, such as vitamins A and E. According to the present invention, various kinds of oily components can be emulsified. Thereamong, it is preferred to use a polar oil, particularly a low-molecular-weight polar oil and, by incorporating it into the oil phase component in an amount of preferably not less than 50% by mass, more preferably not less than 70% by mass, still more preferably not less than 90% by mass, the resulting composition can be imparted with a refreshing feel of use.

Examples of the polar oils include, as synthetic ester oils: isopropyl myristate, cetyl octanoate, octyldodecyl myristate, isopropyl palmitate, butyl stearate, hexyl laurate, myristyl myristate, decyl oleate, hexyldecyl dimethyloctanoate, cetyl lactate, myristyl lactate, lanolin acetate, isocetyl stearate, isocetyl isostearate, ethyl isostearate, cholesteryl 12-hydroxystearate, ethylene glycol di-2-ethylhexanoate, dipentaerythritol fatty acid ester, *N*-alkyl glycol monoisostearate, neopentyl glycol dicaprate, diisostearyl malate, glyceryl di-2-heptylundecanoate, trimethylolpropane tri-2-ethylhexylate, trimethylolpropane triisostearate, pentaerythritol tetra-2-ethylhexylate, glyceryl tri-2-ethylhexylate, and trimethylolpropane triisostearate.

Examples of the polar oils also include cetyl 2-ethyl hexanoate, 2-ethylhexyl palmitate, glycerin trimyristate, tri-2-heptyl undecanoic acid glyceride, castor oil fatty acid methyl ester, oleyl oleate, cetostearyl alcohols, acetoglyceride, 2-heptylundecyl palmitate, diisopropyl adipate, 2-octyldodecyl *N*-lauroyl-*L*-glutamate, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, ethyl acetate, butyl acetate, amyl acetate, triethyl citrate, and octyl methoxycinnamate.

Further, examples of the natural oils include avocado oil, tsubaki oil, turtle fatty acid, macadamia nut oil, corn oil, mink oil, olive oil, rapeseed oil, egg yolk oil, sesame oil, persic oil, wheat germ oil, camellia oil, castor oil, linseed oil, safflower oil, cotton seed oil, perilla oil, soybean oil, peanut oil, tea seed oil, Japanese nutmeg oil, rice bran oil, Chinese tung oil, Japanese tung oil, jojoba oil, germ oil, glyceryl trioctanoate, glyceryl triisopalmitate, and glyceryl tri(caprylate/caprate).

Examples of the hydrocarbon oils include isododecane, isohexadecane, squalane, vaseline, hydrogenated poly(C6-12 olefin), and hydrogenated polyisobutene.

Examples of the non-polar silicone oils include cyclopentasiloxane, decamethylcyclopentasiloxane, caprylyl methicone, dimethicone, (dimethicone/vinyl dimethicone) cross-polymer, and (dimethicone/phenylvinyl dimethicone) cross-polymer.

Examples of the polar silicone oils include diphenylsiloxyphenyl trimethicone.

In the present invention, the oil phase component is added such that the content thereof is preferably 3 to 75% by mass, more preferably 5 to 20% by mass, still more preferably 5 to 15% by mass, with respect to the whole composition. By controlling the content of the oil phase component in this range, a composition having excellent temporal and temperature stability can be produced, and a skin external preparation which yields a good feeling of use can thus be obtained. Particularly, since the ratio of the oil phase (internal phase ratio) with respect to the aqueous phase in the resulting oil-in-water emulsion type composition can be increases, for example, by emulsifying a polar oil, particularly a low-molecular-weight polar oil, at a high internal phase ratio, it is also possible to obtain a skin external preparation which has both refreshing feel and sufficient moisture retention.

### <Aqueous Phase Component>

In the present invention, the aqueous phase component constitutes an external phase of the resulting oil-in-water type emulsion composition.

The aqueous phase component is usually water and a water-soluble component and refers to a component (excluding a surfactant) which is dissolved in water when it is suspended in water at 25 to 65°C and left to stand for 1 hour. The water used in the step of forming an intermediate phase also constitutes an aqueous phase (external phase); however, in the step of adding an aqueous phase component according to the present invention, other aqueous phase component such as water is used.

### <Other Components>

In the composition of the present invention, other optional components can also be incorporated as long as they do not impair the effects of the composition.

Such optional components are not particularly restricted as long as they can be normally incorporated into a skin external preparation, and examples thereof include polyhydric alcohols, surfactants (e.g., cationic surfactants, anionic surfactants, non-ionic surfactants, and silicone-based surfactants), various active ingredients, moisturizers, pH-adjusting agents, thickening agents, preservatives, powders, organic modified clay minerals, and antibacterial agents. Preferred examples of the antibacterial agents include natural antibacterial substances, such as caprylyl glycol, glyceryl caprylate, ethylhexylglycerin and caprylhydroxamic acid.

Examples of the active ingredients include moisturizing components, skin-whitening components, wrinkle-improving components, anti-inflammatory components, and extracts derived from animals and plants. Only one of these active ingredients may be incorporated, or two or more thereof may be incorporated in combination.

Among the above-described optional components, it is particularly preferred to incorporate a polyhydric alcohol. By incorporating a polyhydric alcohol, the ability of the intermediate phase to hold and retain the oil phase component is improved, so that the emulsion stability of the composition can be improved.

In the present invention, a polyhydric alcohol is added such that the content thereof is preferably 1 to 30% by mass, more preferably 1 to 20% by mass, still more preferably 1 to 15% by mass, with respect to the whole composition.

When the polyhydric alcohol content is less than 1% by mass, the oil phase component may not be sufficiently retained in the intermediate phase, whereas when the polyhydric alcohol content is higher than 30% by mass, refreshing feel may be hardly obtained when the resulting composition is applied to the skin.

The polyhydric alcohol is not particularly restricted as long as it is a dihydric or higher alcohol; however, a trihydric or higher alcohol is preferred.

Specific examples of the polyhydric alcohol include dihydric alcohols (e.g., ethylene glycol, propylene glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, and octylene glycol); trihydric alcohols (e.g., glycerol and trimethylolpropane); tetrahydric alcohols (e.g., pentaerythritols such as 1,2,6-hexanetriol); pentahydric alcohols (e.g., xylitol); hexahydric alcohols (e.g., sorbitol and mannitol); polyhydric alcohol copolymers (e.g., diethylene glycol, dipropylene glycol, triethylene glycol, polypropylene glycol, tetraethylene glycol, diglycerol, polyethylene glycol, triglycerol, tetraglycerol, and polyglycerol); dihydric alcohol alkyl ethers (e.g., ethylene glycol monomethyl ether, ethylene glycol monoethyl ether, ethylene glycol monobutyl ether, ethylene glycol monophenyl ether, ethylene glycol monohexyl ether, ethylene glycol mono-2-methylhexyl ether, ethylene glycol isoamyl ether, ethylene glycol benzyl ether, ethylene glycol isopropyl ether, ethylene glycol dimethyl ether, ethylene glycol diethyl ether, and ethylene glycol dibutyl ether); divalent alcohol alkyl ethers (e.g., diethylene glycol monomethyl ether, diethylene glycol monoethyl ether, diethylene glycol monobutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, and dipropylene glycol butyl ether); divalent alcohol ether esters (e.g., ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disuccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, and propylene glycol monophenyl ether acetate); glycerol monoalkyl ethers (e.g., chimyl alcohols, selachyl alcohols, and batyl alcohols); sugar alcohols (e.g., sorbitol, maltitol, maltotriose, mannitol, sucrose, erythritol, glucose, fructose, starch amylolysis sugar, maltose, xylitose, and starch amylolysis sugar-reduced alcohols); glysolid; tetrahydrofurfuryl alcohols; POE-tetrahydrofurfuryl alcohols; POP-butyl ether; POP/POE-butyl ether; tripolyoxypropylene glycerol ether; POP-glycerol ether; POP-glycerol ether phosphate; and POP/POE-pentane erythritol ether.

Thereamong, glycerol, diglycerol, dipropylene glycol, polyethylene glycols having a molecular weight of 600 or less are more preferred, and glycerol is particularly preferred.

These optional components can be added in any step of the present invention, or before or after thereof. For example, depending on the solubility of the respective optional components, the optional components can be added together to the system as oil phase components or aqueous phase components in the step of adding an oil phase component or the step of adding an aqueous phase component, or before or after the step of adding an oil phase component or the step of adding an aqueous phase component.

Oil-in-water type emulsion compositions produced by the present invention have excellent temporal and temperature stability since fine emulsified particles are stably dispersed therein. Further, when the compositions are applied to the skin, the compositions yield a refreshing feel without causing filminess and thus exert a good feeling of use.

Therefore, oil-in-water type emulsion compositions produced by the present invention can be preferably used as skin external preparations and particularly preferably used as cosmetics (including quasi drugs).

### <Oil-in-Water Type Emulsion Composition of Present Invention>

The oil-in-water type emulsion composition obtainable by the method of the present invention comprises: a first surfactant; and a second surfactant, wherein the first surfactant forms an L_{α} phase in at least one aqueous solution having a concentration of 40 to 80% by mass, and the second surfactant forms an H₁ phase in at least one aqueous solution having a concentration of 40 to 80% by mass.

As a concrete combination the surfactants, the following embodiments are preferred.
- An oil-in-water type emulsion composition comprising: at least one alkyl glycoside represented by the above-described following Formula (1); and at least one selected from surfactin and salts thereof, mannosylerythritol lipids, polyglycerol fatty acid esters, sophorolipids, polyoxyethylene glyceryl fatty acids, polyethylene glycol difatty acids, and phospholipids.
- An oil-in-water type emulsion composition comprising: arginine dodecylphosphate; and at least one selected from surfactin and salts thereof, mannosylerythritol lipids, polyglycerol fatty acid esters, sophorolipids, polyoxyethylene glyceryl fatty acids, polyethylene glycol difatty acids, and phospholipids.
- An oil-in-water type emulsion composition comprising: acylamino acid lysine and/or a salt thereof; and at least one selected from surfactin and salts thereof, mannosylerythritol lipids, and polyglycerol fatty acid esters.
- An oil-in-water type emulsion composition comprising: a sucrose fatty acid ester; and at least one selected from surfactin and salts thereof, mannosylerythritol lipids, and polyglycerol fatty acid esters.
- An oil-in-water type emulsion composition comprising: a polyglycerol alkyl ether; and at least one selected from surfactin and salts thereof, mannosylerythritol lipids, and polyglycerol fatty acid esters.

Detailed descriptions of these oil-in-water type emulsion compositions conform to the descriptions provided above for the production method according to the first embodiment of the present invention.

It is noted here that, although these oil-in-water type emulsion compositions are preferably produced by the production method according to the first embodiment of the present invention, they may be produced by other method as well.

When producing an oil-in-water type emulsion composition which comprises acylamino acid lysine and/or a salt thereof and at least one selected from surfactin and salts thereof, mannosylerythritol lipids and polyglycerol fatty acid esters by other method, from the standpoints of the emulsion stability and the feeling of use, it is preferred to set the following conditions.

The concentration of the acylamino acid lysine and/or a salt thereof is preferably 0.01 to 5% by mass, more preferably 0.01 to 3% by mass, still more preferably 0.05 to 2% by mass, with respect to the whole composition.

The concentration of the at least one selected from surfactin and salts thereof, mannosylerythritol lipids and polyglycerol fatty acid esters is preferably 0.01 to 10% by mass, more preferably 0.01 to 3% by mass, still more preferably 0.5 to 2% by mass, with respect to the whole composition.

The total concentration of the acylamino acid lysine and/or a salt thereof and the at least one selected from surfactin and salts thereof, mannosylerythritol lipids and polyglycerol fatty acid esters is preferably 0.02 to 11% by mass, more preferably 0.02 to 6% by mass, still more preferably 0.06 to 4% by mass, with respect to the whole composition.

The mass ratio of the acylamino acid lysine and/or a salt thereof and the at least one selected from surfactin and salts thereof, mannosylerythritol lipids and polyglycerol fatty acid esters is preferably 1:9 to 9:1, more preferably 2:8 to 8:2.

The content of an oil phase component is preferably 1 to 75% by mass, more preferably 3 to 50% by mass, still more preferably 5 to 30% by mass, with respect to the whole composition.

The content of a polyhydric alcohol is preferably 1 to 50% by mass, more preferably 10 to 40% by mass, still more preferably 25 to 35% by mass, with respect to the whole composition.

When producing an oil-in-water type emulsion composition which comprises a sucrose fatty acid ester or a polyglycerol alkyl ether and at least one selected from surfactin and salts thereof, mannosylerythritol lipids, and polyglycerol fatty acid esters by other method, from the standpoints of the emulsion stability and the feeling of use, it is preferred to set the following conditions.

The concentration of the sucrose fatty acid ester or polyglycerol alkyl ether is preferably 0.1 to 5% by mass, more preferably 0.1 to 3% by mass, still more preferably 0.5 to 2% by mass, with respect to the whole composition.

The concentration of the at least one selected from surfactin and salts thereof, mannosylerythritol lipids and polyglycerol fatty acid esters is preferably 0.1 to 10% by mass, more preferably 0.3 to 3% by mass, still more preferably 0.5 to 2% by mass, with respect to the whole composition.

The total concentration of the sucrose fatty acid ester or polyglycerol alkyl ether and the at least one selected from surfactin and salts thereof, mannosylerythritol lipids and polyglycerol fatty acid esters is preferably 0.2 to 8% by mass, more preferably 0.5 to 6% by mass, still more preferably 0.6 to 4% by mass, with respect to the whole composition.

The mass ratio of the sucrose fatty acid ester or polyglycerol alkyl ether and the at least one selected from surfactin and salts thereof, mannosylerythritol lipids and polyglycerol fatty acid esters is preferably 1:9 to 9:1, more preferably 2:8 to 8:2.

The content of an oil phase component is preferably 1 to 75% by mass, more preferably 3 to 50% by mass, still more preferably 5 to 30% by mass, with respect to the whole composition.

The content of a polyhydric alcohol is preferably 1 to 50% by mass, more preferably 5 to 35% by mass, still more preferably 8 to 20% by mass, with respect to the whole composition.

### <Composition for Maturation of Skin Horny Layer>

The present inventors also discovered that the oil-in-water type emulsion composition obtainable by the method of the present invention exerts a novel action.

Skin is a tissue which protects a living body from external stimuli and the like and has a barrier function. This barrier function is known to be largely dependent on the state of corneocytes existing in a horny layer, and technologies of improving the barrier function by increasing the area of corneocytes through maturation have been proposed (see Japanese Laid-open Patent Application (Kokai) Nos. 2004-035456 and 2007-014294).

Meanwhile, a horny layer is constituted by corneocytes and their surrounding intercellular lipids. Corneocytes have a cornified envelope, and maturation of the cornified envelope has been investigated (see Japanese Laid-open Patent Application (Kokai) Nos. 2008-303185, 2008-303186 and No. 2008-303187). It is noted here that "corneocyte" is synonymous to "horny layer cell".

Under these circumstances where technologies for maturation of corneocytes and maturation of cornified envelops have been proposed, the present inventors examined a novel technology of allowing a horny layer to maturate by an approach different from conventional ones.

As a result, the present inventors discovered that an oil-in-water type emulsion composition comprising a first surfactant and a second surfactant, wherein the first surfactant forms an L_{α} phase in at least one aqueous solution having a concentration of 40 to 80% by mass and the second surfactant forms an H₁ phase in at least one aqueous solution having a concentration of 40 to 80% by mass, has an action of maturating a skin horny layer.

That is, the oil-in-water type emulsion composition obtainable by the method of the present invention is suitable for applications of maturating a skin horny layer. In other words, the use of the oil-in-water type emulsion composition obtainable by the method of the present invention for maturation of skin horny layer is provided.

Further, a method which comprises the step of applying the oil-in-water type emulsion composition obtainable by the method of the present invention to the skin can be a method of maturating a skin horny layer.

Moreover, the production method of the present invention can also be regarded as a method of preparing an oil-in-water type emulsion composition for maturation of skin horny layer.

The mechanism of horny layer maturation attained by the use of a combination of the above-described two kinds of first and second surfactants as discovered by the present inventors is not clear; however, it is speculated that a hexagonal liquid crystal phase H₁ and a lamellar liquid crystal phase L_{α}, which are formed as intermediate phases in the preparation of the composition, have some effect on horny layers.

The use of a transdermal absorption-promoting component such as a surfactant usually causes some damage to the maintenance of the barrier function of skin horny layer, and it could not be anticipated that the use of the above-described two surfactants in combination would yield a phenomenon of skin horny layer maturation.

Furthermore, the point that the composition obtainable by the method of the present invention has an action of maturating a skin horny layer and thereby exerts a skin moisturizing effect without an addition of a moisturizer is also an effect that is not attained by conventional surfactants.

In the present specification, maturation of a horny layer satisfies one or both of the following two phenomena:
i) lamination of horny layers
ii) enucleation

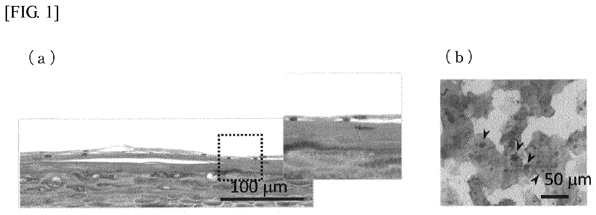

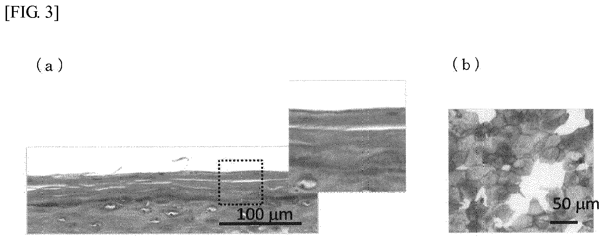

The i) lamination of horny layers can be confirmed by a micrograph of tape-stripped horny layer cells. In the untreated human epidermis model shown in FIG. 1(a), the horny layer in the upper part of the micrograph is in a very thin state. In FIG. 3(a) showing the human epidermis model after the application of an H₁ surfactant and an L_{α} surfactant, however, it is understood that lamination of horny layers had advanced in the upper part of the micrograph.

Specifically, lamination of horny layers may be defined as a case where the number of horny layers increases over a period of 24 hours after the application. In this case, the number of horny layers may be not less than 1.1 times, not less than 1.2 times, not less than 1.25 times, not less than 1.3 times, or not less than 1.5 times, as compared to the number of horny layers prior to the application.

Alternatively, lamination of horny layers may be defined as a case where the thickness of a horny layer increased over a period of 24 hours after the application. In this case, the thickness of the horny layer may be not less than 1.1 times, not less than 1.2 times, not less than 1.25 times, not less than 1.3 times, or not less than 1.5 times, as compared to the thickness of the horny layer prior to the application.

The ii) enucleation can be confirmed by a cross-sectional micrograph of tape-stripped horny layer cells. In the untreated human epidermis model shown in FIG. 1(b), it is understood that a large number of nuclei exist in the horny layer. In FIG. 3(b) showing the human epidermis model after the application of an H₁ surfactant and an L_{α} surfactant, however, it is understood that the nuclei in the horny layer had disappeared. Specifically, enucleation may be defined as a case where the number of nuclei in a horny layer decreases over a period of 24 hours after the application. In this case, the number of visible nuclei in the horny layer may decrease to 50% or less, 25% or less or 10% or less, or the visible nuclei completely disappear.

### EXAMPLES

The present invention will now be described in more detail by way of Examples thereof; however, the present invention is not restricted to the following Examples as long as they do not deviate from the gist of the present invention, as defined in the claims.

### <Reference Experimental Example 1>

As first surfactants, 60%-by-mass aqueous solutions of surfactin sodium, MEL-B, ACS-Sophor (manufactured by Allied Carbon Solutions Co., Ltd.), polyoxyethylene (20) glyceryl triisostearate, PEG-12 diisostearate, polyglyceryl-5 oleate, polyglyceryl-6 laurate and hydrogenated lecithin were each prepared. In addition, as second surfactants, 60%-by-mass aqueous solutions of Mal₂Far, Mal₃Far, Mal₃Phyt, sucrose laurate, lysine dilauroyl glutamate, polyglyceryl-4 monolauryl ether, polyglyceryl-10 monolauryl ether, lauroyl maltoside and arginine dodecylphosphate were each prepared.

These aqueous solutions (25°C, except for hydrogenated lecithin at 60°C) were subjected to X-ray diffraction analysis to confirm the formation of an L_{α} phase and the formation of an H₁ phase in the aqueous solutions of the first surfactants and the aqueous solutions of the second surfactants, respectively.

### <Experimental Example 1> (not prepared according to the claimed method)

The oil-in-water type emulsion compositions of Examples 1 to 11 and Comparative Examples 1 to 6 were each prepared in accordance with the formulations shown in Table 1. Specifically, to the component A which had been heated to 80°C and homogenized, the components B, C, D and E each heated to 80°C were sequentially added with manual stirring, and the resultant was subsequently cooled to 30°C with stirring.

### [Table 1]

**Table 1 (% by mass)**

| | Component name | Example 1 | Example 2 | Example 3 | Example 4 | Example 5 | Example 6 | Example 7 | Example 8 | Example 9 | Example 10 | Example 11 | Comparative Example 1 | Comparative Example 2 | Comparative Example 3 | Comparative Example 4 | Comparative Example 5 | Comparative Example 6 |
|---|---|---|---|---|---|---|---|---|---|---|---|---|---|---|---|---|---|---|
| A | PEG-12 diisostearate | 0.5 | 0.5 | 0.8 | 0.8 | 0.8 | | | | | | | 1.0 | | | | 0.5 | |
| | arginine dodecylphosphate | 0.5 | 0.5 | 0.5 | 0.2 | 0.2 | | | | | | | | 1.0 | | | 0.5 | |
| | polyglyceryl-5 oleate | | | | | | 0.5 | 0.5 | 0.2 | 0.5 | 0.5 | 0.8 | | | 1.0 | | | |
| | Mal2Far* | | | | | | 0.5 | 0.5 | 0.8 | 0.5 | 0.5 | 0.2 | | | | 1.0 | | |
| | hydrogenated lecithin | | | | | | | | | | | | | | | | | 2.0 |
| | water | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 | 1.0 |
| | glycerol | 10.0 | 10.0 | 10.0 | 10.0 | 10.0 | 10.0 | 10.0 | 10.0 | 10.0 | 10.0 | 10.0 | 10.0 | 10.0 | 10.0 | 10.0 | 10.0 | 20.0 |
| | BG | | | | | | | | | | | | | | | | 3.0 | |
| | xanthan gum | | | | | | | | | | | | | | | | 0.1 | |
| | methylparaben | | | | | | | | | | | | | | | | 0.2 | |
| | phenoxyethanol | | | | | | | | | | | | | | | | 0.3 | |
| B | squalane | 20.0 | | 35.0 | 20.0 | | 20.0 | 8.0 | | | | | | 20.0 | | | | 20.0 |
| | glyceryl tri-2-ethylhexylate | | 10.0 | | | | | | 20.0 | | 10.0 | | 20.0 | | | | 20.0 | |
| | glyceryl tri(caprylate/caprate) | | | | | 20.0 | | | | 20.0 | | | | | 20.0 | | | |
| | ethyl isostearate | | | | | | | | | | | 20.0 | | | | 20.0 | | |
| C | xanthan gum | 0.3 | 0.1 | 0.1 | 0.1 | 0.1 | 0.1 | 0.1 | 0.1 | 0.1 | 0.1 | 0.1 | 0.3 | 0.1 | 0.1 | 0.1 | 0.1 | 0.1 |
| | agar | 0.3 | | | | | | | | | | | 0.3 | | | | | |
| | methylparaben | | 0.2 | 0.2 | 0.2 | 0.2 | 0.2 | 0.2 | 0.2 | 0.2 | 0.2 | 0.2 | | 0.2 | 0.2 | 0.2 | 0.2 | 0.2 |
| | phenoxyethanol | | 0.3 | 0.3 | 0.3 | 0.3 | 0.3 | 0.3 | 0.3 | 0.3 | 0.3 | 0.3 | | 0.3 | 0.3 | 0.3 | 0.3 | 0.3 |
| | propanediol | 5.0 | | | | | | | | | | | 5.0 | | | | | |
| | BG | | 3.0 | 3.0 | 3.0 | 3.0 | 3.0 | 3.0 | 3.0 | 3.0 | 3.0 | 3.0 | | 3.0 | 3.0 | 3.0 | 3.0 | 3.0 |
| | caprylyl glycol | 0.05 | | | | | | | | 0.05 | 0.05 | | 0.05 | | | | | |
| | glyceryl caprylate | 0.05 | | | | | | | | 0.05 | 0.05 | | 0.05 | | | | | |
| | ethylhexylglycerin | 0.05 | | | | | | | | 0.05 | 0.05 | | 0.05 | | | | | |
| | caprylhydroxamic acid | 0.05 | | | | | | | | 0.05 | 0.05 | | 0.05 | | | | | |
| | water | to 100 | to 100 | to 100 | to 100 | to 100 | to 100 | to 100 | to 100 | to 100 | to 100 | to 100 | to 100 | to 100 | to 100 | to 100 | to 100 | to 100 |
| D | carbomer | | 0.10 | 0.10 | 0.10 | 0.10 | 0.10 | 0.10 | 0.10 | 0.10 | 0.10 | 0.10 | | 0.10 | 0.10 | 0.10 | 0.10 | 0.10 |
| | water | | 10.0 | 10.0 | 10.0 | 10.0 | 10.0 | 10.0 | 10.0 | 10.0 | 10.0 | 10.0 | | 10.0 | 10.0 | 10.0 | 10.0 | 10.0 |
| E | potassium hydroxide | | 0.07 | 0.07 | 0.07 | 0.07 | 0.07 | 0.07 | 0.07 | 0.07 | 0.07 | 0.07 | | 0.07 | 0.07 | 0.07 | 0.07 | 0.07 |
| | water | | 0.63 | 0.63 | 0.63 | 0.63 | 0.63 | 0.63 | 0.63 | 0.63 | 0.63 | 0.63 | | 0.63 | 0.63 | 0.63 | 0.63 | 0.63 |

| | | | | | | | | | | | | | | | | | | |
|---|---|---|---|---|---|---|---|---|---|---|---|---|---|---|---|---|---|---|
| *Mal2Far: glycoside of maltose and hexahydrofarnesol (1-O-3,7,11-trimethyldodecyl)-*β*-D-maltoside) | | | | | | | | | | | | | | | | | | |

In addition, the oil-in-water type emulsion composition of Example 12 was prepared in accordance with the formulation shown in Table 2. Specifically, the components A and B, which had been each heated to 80°C and homogenized, were mixed and homogenized with manual stirring, and then the components C and D each heated to 80°C were sequentially added thereto, after which the resultant was cooled to 30°C with stirring.

### [Table 2]

**Table 2**

| (% by mass) | | |
|---|---|---|
| | Component name | Example 12 |
| A | polyglyceryl-5 oleate | 0.5 |
| | water | 5.0 |
| B | Mal₂Far^{∗} | 0.5 |
| | water | 5.0 |
| C | squalane | 20.0 |
| D | xanthan gum | 0.3 |
| | agar | 0.3 |
| | propanediol | 5.0 |
| | glycerol | 10.0 |
| | caprylyl glycol | 0.05 |
| | glyceryl caprylate | 0.05 |
| | ethylhexylglycerin | 0.05 |
| | caprylhydroxamic acid | 0.05 |
| | water | to 100 |

| | | |
|---|---|---|
| ^{∗}Mal₂Far: glycoside of maltose and hexahydrofarnesol (1-O-3,7,11-trimethyldodecyl)-*β*-D-maltoside) | | |

Further, the oil-in-water type emulsion composition of Comparative Example 7 was prepared in accordance with the formulation shown in Table 3. Specifically, the component B heated to 80°C was added with manual stirring to the component A which had been heated to 80°C and homogenized, and the resultant was cooled to 30°C with stirring.

### [Table 3]

**Table 3**

| (% by mass) | | |
|---|---|---|
| | Component name | Comparative Example 7 |
| A | polyglyceryl-5 oleate | 0.5 |
| | Mal₂Far^{∗} | 0.5 |
| | water | to 100 |
| | glycerol | 10.0 |
| | BG | 3.0 |
| | xanthan gum | 0.1 |
| | methylparaben | 0.2 |
| | phenoxyethanol | 0.3 |
| | carbomer | 0.10 |
| | potassium hydroxide | 0.07 |
| B | glyceryl tri-2-ethylhexylate | 20.0 |

| | | |
|---|---|---|
| ^{∗}Mal₂Far: glycoside of maltose and hexahydrofarnesol (1-O-3,7,11-trimethyldodecyl)-*β*-D-maltoside) | | |

### <Test Example 1>

### Evaluation of Size of Emulsified Particles

The oil-in-water type emulsion compositions of Examples and Comparative Examples were each left to stand at 20°C for one day after the preparation and then observed under a light microscope (OLYMPUS BX51) to evaluate the size and uniformity of their emulsified particles based on the following four criteria. The results thereof are shown in Table 4.
⊚: The emulsified particles were small (1 µm or smaller) and uniform.
∘: The emulsified particles were small (1 µm or smaller), but not uniform.
Δ: The emulsified particles were large (larger than 1 µm), but uniform.
×: The emulsified particles were large (larger than 1 µm) and not uniform.

### <Test Example 2>

### Evaluation of Temporal and Temperature Stability

The oil-in-water type emulsion compositions of Examples and Comparative Examples were each left to stand at 40°C for one month after the preparation. Then, the presence or absence of changes in state, such as phase separation and creaming, was visually observed, and the emulsified particles were observed under a light microscope in the same manner as in Test Example 1 and compared with the emulsified particles of immediately after the preparation. The results thereof are shown in Table 4.

### <Test Example 3>

### Evaluation of Feeling of Use

Twenty female subjects were asked to use each of the oil-in-water type emulsion compositions of Examples and Comparative Examples on their skin and to evaluate the feeling of use on a scale of 1 to 5 as follows. Table 4 shows the evaluation results in terms of average score given by the 20 subjects.
5: moderately refreshing
4: slightly refreshing
3: slightly sticky
2: sticky
1: very sticky

### <Test Example 4>

The oil-in-water type emulsion compositions of Examples and Comparative Examples were each left to stand at 20°C for one day after the preparation, and the viscosity was measured using a B-type viscometer (DIGITAL VISMETRON VDA, manufactured by Shibaura Systems Co., Ltd.) with a rotor No. 3 at a measurement temperature of 20°C and a rotation rate of 6 rpm for 60 seconds. The results thereof are shown in Table 4. Generally speaking, the tendency is that the lower the viscosity of an emulsion composition, the harder it is to maintain the stability; however, it is seen that the oil-in-water type emulsion compositions produced by the production method of the present invention have excellent temporal stability even at a low viscosity of about 3,000 to 4,000.

### [Table 4]

**Table 4**

| | Example 1 | Example 2 | Example 3 | Example 4 | Example 5 | Example 6 | Example 7 | Example 8 | Example 9 | Example 10 | Example 11 | Example 12 | Comparative Example 1 | Comparative Example 2 | Comparative Example 3 | Comparative Example 4 | Comparative Example 5 | Comparative Example 6 | Comparative Example 7 |
|---|---|---|---|---|---|---|---|---|---|---|---|---|---|---|---|---|---|---|---|
| Evaluation of size of emulsified particles | ⊚ | ⊚ | ⊚ | ∘ | ∘ | ⊚ | ⊚ | ∘ | ⊚ | ⊚ | ∘ | ⊚ | x | Δ | x | x | x | ∘ | x |
| Viscosity (mP·a)/20°C, after 1 day | 3,800 | 3,500 | 8,000 | 4,200 | 4,400 | 4,600 | 3,800 | 3,600 | 4,000 | 3,500 | 3,600 | 3,800 | 4,200 | 3,800 | 4,800 | 5,000 | 3,400 | 10,000 | 3,400 |
| Evaluation of feeling of use | 4 | 5 | 3 | 4 | 4 | 3 | 5 | 4 | 4 | 5 | 5 | 4 | 3 | 3 | 2 | 3 | 4 | 1 | 4 |
| Temporal stability (40°C, after 1 month) | stable | stable | stable | stable | stable | stable | stable | stable | stable | stable | stable | stable | slight creaming | slight creaming | creaming | slight creaming | creaming | stable | creaming |

### <Experimental Example 2> (not prepared according to the claimed method)

The oil-in-water type emulsion compositions of Examples and Comparative Examples were prepared in accordance with the formulations shown in Tables 5 to 11. Specifically, the components A, B and C were each heated to 80°C and homogenized, and the components A and C were mixed. After adding the component B thereto, the resultant was cooled to 30°C with stirring.

The thus prepared compositions were each evaluated in the same manner as in Test Examples 1 to 3. The results thereof are shown in each Table below.

### [Table 5]

**Table 5**

| (% by mass) | | | | | | | | | | | | | | | | | | |
|---|---|---|---|---|---|---|---|---|---|---|---|---|---|---|---|---|---|---|
| Component name | | Example 13 | Example 14 | Example 15 | Example 16 | Example 17 | Example 18 | Example 19 | Example 20 | Example 21 | Example 22 | Example 23 | Example 24 | Comparative Example 8 | Comparative Example 9 | Comparative Example 10 | Comparative Example 11 | Comparative Example 12 |
| A | sodium dilauramidoglutamide lysine | 0.2 | 0.2 | 0.2 | 0.2 | 0.2 | 0.2 | 0.2 | 0.2 | 0.2 | 0.5 | 0.2 | 0.03 | | | | | 1 |
| | MEL-B | 0.8 | | | | | | | 0.8 | 0.8 | 0.5 | 0.8 | 0.97 | 1 | | | 0.5 | |
| | surfactin Na | | 0.8 | | | | | | | | | | | | 1 | | 0.5 | |
| | polyglyceryl-5 monooleate | | | 0.8 | | | | | | | | | | | | 1 | | |
| | polyglyceryl-4 monolaurate | | | | 0.8 | | | | | | | | | | | | | |
| | polyglyceryl-6 monolaurate | | | | | 0.8 | | | | | | | | | | | | |
| | polyglyceryl-6 monopalmitate | | | | | | 0.8 | | | | | | | | | | | |
| | polyglyceryl-6 dilaurate | | | | | | | 0.8 | | | | | | | | | | |
| B | squalane | 10 | | | 10 | 10 | | 10 | 15 | | 10 | 4 | 10 | 10 | | | | 10 |
| | glyceryl | | 10 | 10 | | | | | | 10 | | 7 | | | 10 | | 10 | |
| | tri(caprylate/caprate) | | | | | | | | | | | | | | | | | |
| | triethylhexanoin | | | | | | 10 | | 15 | | 10 | 9 | | | | 10 | | |
| C | methylparaben | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 | 0.2 |
| | phenoxyethanol | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 | 0.5 |
| | xanthan gum | 0.05 | 0.05 | 0.05 | 0.05 | 0.05 | 0.05 | 0.05 | 0.05 | 0.05 | 0.05 | 0.05 | 0.05 | 0.05 | 0.05 | 0.05 | 1.05 | 3.05 |
| | carbomer | 0.1 | 0.1 | 0.1 | 0.1 | 0.1 | 0.1 | 0.1 | 0.1 | 0.1 | 0.1 | 0.1 | 0.1 | 0.1 | 0.1 | 0.1 | 1.1 | 3.1 |
| | glycerol | 8 | 8 | 8 | 8 | 8 | 8 | 8 | 20 | 8 | 20 | 8 | 8 | 8 | 8 | 8 | 20 | 8 |
| | BG | 5 | 5 | 5 | 5 | 5 | 5 | 5 | 5 | 5 | 5 | 5 | 5 | 5 | 5 | 5 | 5 | 5 |
| | potassium hydroxide | 0.07 | 0.07 | 0.07 | 0.07 | 0.07 | 0.07 | 0.07 | 0.07 | 0.07 | 0.07 | 0.07 | 0.07 | 0.07 | 0.07 | 0.07 | 0.07 | 0.07 |
| | water | to 100 | to 100 | to 100 | to 100 | to 100 | to 100 | to 100 | to 100 | to 100 | to 100 | to 100 | to 100 | to 100 | to 100 | to 100 | to 101 | to 103 |
| Evaluation results | Size of emulsified particles | ⊚ | ⊚ | ∘ | c | ⊚ | ∘ | ∘ | ⊚ | ⊚ | ⊚ | ∘ | ∘ | Δ | ∘ | x | Δ | Δ |
| | Temporal stability (at 40°C, after 1 month) | stable | stable | stable | stable | stable | stable | stable | stable | stable | stable | stable | stable | slight creaming | stable | creaming | slight creaming | slight creaming |
| | Usability (stickiness) | 4 | 5 | 3 | 4 | 4 | 5 | 4 | 3 | 4 | 3 | 3 | 3 | 3 | 2 | 2 | 2 | 2 |
| | Usability (flexibility) | 3 | 3 | 3 | 3 | 3 | 3 | 3 | 5 | 3 | 4 | 4 | 3 | 3 | 1 | 3 | 2 | 1 |

### [Table 6]

**Table 6**

| | Component name | Example 25 | Example 26 | Example 27 | Example 28 | Example 29 | Example 30 | Example 31 |
|---|---|---|---|---|---|---|---|---|
| A | sucrose laurate | 0.50 | 0.50 | 0.50 | 0.50 | 0.50 | | 0.10 |
| | sucrose stearate | | | | | | 0.50 | |
| | surfactin sodium | 0.50 | | | | | 0.50 | 0.10 |
| | MEL-B | | 0.50 | | | | | |
| | polyglyceryl-4 monolaurate | | | 0.50 | | | | |
| | polyglyceryl-10 monolaurate | | | | 0.50 | | | |
| | polyglyceryl-5 monooleate | | | | | 0.50 | | |
| | POE (25) stearate | | | | | | | |
| B | squalane | 3.00 | 3.00 | 3.00 | 3.00 | 3.00 | 3.00 | 3.00 |
| | glyceryl tri(caprylate/caprate) | 5.00 | 5.00 | 5.00 | 5.00 | 5.00 | 5.00 | 5.00 |
| | triethylhexanoin | 7.00 | 7.00 | 7.00 | 7.00 | 7.00 | 7.00 | 7.00 |
| C | methylparaben | 0.20 | 0.20 | 0.20 | 0.20 | 0.20 | 0.20 | 0.20 |
| | phenoxy ethanol | 0.50 | 0.50 | 0.50 | 0.50 | 0.50 | 0.50 | 0.50 |
| | xanthan gum | 0.05 | 0.05 | 0.05 | 0.05 | 0.05 | 0.05 | 0.05 |
| | carbomer | 0.10 | 0.10 | 0.10 | 0.10 | 0.10 | 0.10 | 0.10 |
| | glycerol | 8.00 | 8.00 | 8.00 | 8.00 | 8.00 | 8.00 | 8.00 |
| | 1,3-butylene glycol | 5.00 | 5.00 | 5.00 | 5.00 | 5.00 | 5.00 | 5.00 |
| | potassium hydroxide | 0.07 | 0.07 | 0.07 | 0.07 | 0.07 | 0.07 | 0.07 |
| | water | residual amount | residual amount | residual amount | residual amount | residual amount | residual amount | residual amount |
| Evaluation results | Size of emulsified particles | ⊚ | ⊚ | ⊚ | ⊚ | ⊚ | ∘ | ∘ |
| | Temporal stability (at 40°C, after 1 month) | stable | stable | stable | stable | stable | stable | stable |
| | Usability (stickiness) | 5 | 4 | 4 | 4 | 4 | 4 | 4 |
| | Usability (flexibility) | 5 | 5 | 5 | 4 | 5 | 4 | 3 |

| | | | | | | | | |
|---|---|---|---|---|---|---|---|---|
| (% by mass) | | | | | | | | |

### [Table 7]

**Table 7**

| | Component name | Example 32 | Example 33 | Example 34 | Example 35 | Example 36 | Example 37 | Example 38 |
|---|---|---|---|---|---|---|---|---|
| A | sucrose laurate | 0.05 | 5.00 | 0.50 | 0.50 | 0.10 | 0.10 | 0.50 |
| | sucrose stearate | | | | | | | |
| | surfactin sodium | 0.05 | 1.00 | 0.50 | 0.50 | 0.90 | 1.00 | 0.50 |
| | MEL-B | | | | | | | |
| | polyglyceryl-4 monolaurate | | | | | | | |
| | polyglyceryl-10 monolaurate | | | | | | | |
| | polyglyceryl-5 monooleate | | | | | | | |
| | POE (25) stearate | | | | | | | |
| B | squalane | 3.00 | 3.00 | 9.00 | 3.00 | 3.00 | 3.00 | 10.00 |
| | glyceryl tri(caprylate/caprate) | 5.00 | 5.00 | 15.00 | 5.00 | 5.00 | 5.00 | 3.00 |
| | triethylhexanoin | 7.00 | 7.00 | 21.00 | 7.00 | 7.00 | 7.00 | 2.00 |
| c | methylparaben | 0.20 | 0.20 | 0.20 | 0.20 | 0.20 | 0.20 | 0.20 |
| | phenoxy ethanol | 0.50 | 0.50 | 0.50 | 0.50 | 0.50 | 0.50 | 0.50 |
| | xanthan gum | 0.05 | 0.05 | 0.05 | 0.05 | 0.05 | 0.05 | 0.05 |
| | carbomer | 0.10 | 0.10 | 0.10 | 0.10 | 0.10 | 0.10 | 0.10 |
| | glycerol | 8.00 | 8.00 | 8.00 | 25.00 | 8.00 | 8.00 | 8.00 |
| | 1,3-butylene glycol | 5.00 | 5.00 | 5.00 | 10.00 | 5.00 | 5.00 | 5.00 |
| | potassium hydroxide | 0.07 | 0.07 | 0.07 | 0.07 | 0.07 | 0.07 | 0.07 |
| | water | residual amount | residual amount | residual amount | residual amount | residual amount | residual amount | residual amount |
| Evaluation results | Size of emulsified particles | Δ | ⊚ | ⊚ | ⊚ | ⊚ | ∘ | ⊚ |
| | Temporal stability (at 40°C, after 1 month) | stable | stable | stable | stable | stable | stable | stable |
| | Usability (stickiness) | 3 | 3 | 3 | 3 | 4 | 4 | 5 |
| | Usability (flexibility) | 3 | 3 | 5 | 4 | 3 | 3 | 5 |

| | | | | | | | | |
|---|---|---|---|---|---|---|---|---|
| (% by mass) | | | | | | | | |

### [Table 8]

**Table 8**

| | Component name | Comparative Example 13 | Comparative Example 14 | Comparative Example 15 |
|---|---|---|---|---|
| A | sucrose laurate | | 1.00 | 0.50 |
| | sucrose stearate | | | |
| | surfactin sodium | 1.00 | | |
| | MEL-B | | | |
| | polyglyceryl-4 monolaurate | | | |
| | polyglyceryl-10 monolaurate | | | |
| | polyglyceryl-5 monooleate | | | |
| | POE (25) stearate | | | 0.50 |
| B | squalane | 3.00 | 3.00 | 3.00 |
| | glyceryl tri(caprylate/caprate) | 5.00 | 5.00 | 5.00 |
| | triethylhexanoin | 7.00 | 7.00 | 7.00 |
| C | methylparaben | 0.20 | 0.20 | 0.20 |
| | phenoxy ethanol | 0.50 | 0.50 | 0.50 |
| | xanthan gum | 0.05 | 0.05 | 0.05 |
| | carbomer | 0.10 | 0.10 | 0.10 |
| | glycerol | 8.00 | 8.00 | 8.00 |
| | 1,3-butylene glycol | 5.00 | 5.00 | 5.00 |
| | potassium hydroxide | 0.07 | 0.07 | 0.07 |
| | water | residual amount | residual amount | residual amount |
| Evaluation results | Size of emulsified particles | Δ | × | Δ |
| | Temporal stability (at 40°C, after 1 month) | unstable | unstable | unstable |
| | Usability (stickiness) | 3 | 2 | 2 |
| | Usability (flexibility) | 2 | 2 | 1 |

| | | | | |
|---|---|---|---|---|
| (% by mass) | | | | |

### [Table 9]

**Table 9**

| | Component name | Example 39 | Example 40 | Example 41 | Example 42 | Example 43 | Example 44 | Example 45 |
|---|---|---|---|---|---|---|---|---|
| A | polyglycerol-4 monolauryl ether | 0.50 | 0.50 | 0.50 | 0.50 | 0.50 | | 0.10 |
| | polyglycerol-10 monolauryl ether | | | | | | 0.50 | |
| | surfactin sodium | 0.50 | | | | | 0.50 | 0.10 |
| | MEL-B | | 0.50 | | | | | |
| | polyglyceryl-4 monolaurate | | | 0.50 | | | | |
| | polyglyceryl-10 monolaurate | | | | 0.50 | | | |
| | polyglyceryl-5 monooleate | | | | | 0.50 | | |
| | POE (25) stearate | | | | | | | |
| B | squalane | 3.00 | 3.00 | 3.00 | 3.00 | 3.00 | 3.00 | 3.00 |
| | glyceryl tri(caprylate/caprate) | 5.00 | 5.00 | 5.00 | 5.00 | 5.00 | 5.00 | 5.00 |
| | triethylhexanoin | 7.00 | 7.00 | 7.00 | 7.00 | 7.00 | 7.00 | 7.00 |
| c | methylparaben | 0.20 | 0.20 | 0.20 | 0.20 | 0.20 | 0.20 | 0.20 |
| | phenoxy ethanol | 0.50 | 0.50 | 0.50 | 0.50 | 0.50 | 0.50 | 0.50 |
| | xanthan gum | 0.05 | 0.05 | 0.05 | 0.05 | 0.05 | 0.05 | 0.05 |
| | carbomer | 0.10 | 0.10 | 0.10 | 0.10 | 0.10 | 0.10 | 0.10 |
| | glycerol | 8.00 | 8.00 | 8.00 | 8.00 | 8.00 | 8.00 | 8.00 |
| | 1,3-butylene glycol | 5.00 | 5.00 | 5.00 | 5.00 | 5.00 | 5.00 | 5.00 |
| | potassium hydroxide | 0.07 | 0.07 | 0.07 | 0.07 | 0.07 | 0.07 | 0.07 |
| | water | residual amount | residual amount | residual amount | residual amount | residual amount | residual amount | residual amount |
| Evaluation results | Size of emulsified particles | ⊚ | ⊚ | ⊚ | ⊚ | ⊚ | ∘ | ∘ |
| | Temporal stability (at 40°C, after 1 month) | stable | stable | stable | stable | stable | stable | stable |
| | Usability (stickiness) | 5 | 4 | 4 | 4 | 4 | 4 | 4 |
| | Usability (flexibility) | 5 | 5 | 5 | 4 | 5 | 4 | 3 |

| | | | | | | | | |
|---|---|---|---|---|---|---|---|---|
| (% by mass) | | | | | | | | |

### [Table 10]

**Table 10**

| | Component name | Example 46 | Example 47 | Example 48 | Example 49 | Example 50 | Example 51 | Example 52 |
|---|---|---|---|---|---|---|---|---|
| A | polyglycerol-4 monolauryl ether | 0.05 | 5.00 | 0.50 | 0.50 | 0.10 | 0.10 | 0.50 |
| | polyglycerol-10 monolauryl ether | | | | | | | |
| | surfactin sodium | 0.05 | 1.00 | 0.50 | 0.50 | 0.90 | 1.00 | 0.50 |
| | MEL-B | | | | | | | |
| | polyglyceryl-4 monolaurate | | | | | | | |
| | polyglyceryl-10 monolaurate | | | | | | | |
| | polyglyceryl-5 monooleate | | | | | | | |
| | POE (25) stearate | | | | | | | |
| B | squalane | 3.00 | 3.00 | 9.00 | 3.00 | 3.00 | 3.00 | 10.00 |
| | glyceryl tri(caprylate/caprate) | 5.00 | 5.00 | 15.00 | 5.00 | 5.00 | 5.00 | 3.00 |
| | triethylhexanoin | 7.00 | 7.00 | 21.00 | 7.00 | 7.00 | 7.00 | 2.00 |
| c | methylparaben | 0.20 | 0.20 | 0.20 | 0.20 | 0.20 | 0.20 | 0.20 |
| | phenoxy ethanol | 0.50 | 0.50 | 0.50 | 0.50 | 0.50 | 0.50 | 0.50 |
| | xanthan gum | 0.05 | 0.05 | 0.05 | 0.05 | 0.05 | 0.05 | 0.05 |
| | carbomer | 0.10 | 0.10 | 0.10 | 0.10 | 0.10 | 0.10 | 0.10 |
| | glycerol | 8.00 | 8.00 | 8.00 | 25.00 | 8.00 | 8.00 | 8.00 |
| | 1,3-butylene glycol | 5.00 | 5.00 | 5.00 | 10.00 | 5.00 | 5.00 | 5.00 |
| | potassium hydroxide | 0.07 | 0.07 | 0.07 | 0.07 | 0.07 | 0.07 | 0.07 |
| | water | residual amount | residual amount | residual amount | residual amount | residual amount | residual amount | residual amount |
| Evaluation results | Size of emulsified particles | Δ | ⊚ | ⊚ | ⊚ | ⊚ | ∘ | ⊚ |
| | Temporal stability (at 40°C, after 1 month) | stable | stable | stable | stable | stable | stable | stable |
| | Usability (stickiness) | 3 | 3 | 3 | 3 | 4 | 4 | 5 |
| | Usability (flexibility) | 3 | 4 | 5 | 4 | 3 | 3 | 5 |

| | | | | | | | | |
|---|---|---|---|---|---|---|---|---|
| (% by mass) | | | | | | | | |

[Table 11]

**Table 11**

| | Component name | Comparative Example 16 | Comparative Example 17 | Comparative Example 18 |
|---|---|---|---|---|
| A | polyglycerol-4 monolauryl ether | | 1.00 | 0.50 |
| | polyglycerol-10 monolauryl ether | | | |
| | surfactin sodium | 1.00 | | |
| | MEL-B | | | |
| | polyglyceryl-4 monolaurate | | | |
| | polyglyceryl-10 monolaurate | | | |
| | polyglyceryl-5 monooleate | | | |
| | POE (25) stearate | | | 0.50 |
| B | squalane | 3.00 | 3.00 | 3.00 |
| | glyceryl tri(caprylate/caprate) | 5.00 | 5.00 | 5.00 |
| | triethylhexanoin | 7.00 | 7.00 | 7.00 |
| C | methylparaben | 0.20 | 0.20 | 0.20 |
| | phenoxy ethanol | 0.50 | 0.50 | 0.50 |
| | xanthan gum | 0.05 | 0.05 | 0.05 |
| | carbomer | 0.10 | 0.10 | 0.10 |
| | glycerol | 8.00 | 8.00 | 8.00 |
| | 1,3-butylene glycol | 5.00 | 5.00 | 5.00 |
| | potassium hydroxide | 0.07 | 0.07 | 0.07 |
| | water | residual amount | residual amount | residual amount |
| Evaluation results | Size of emulsified particles | Δ | Δ | Δ |
| | Temporal stability (at 40°C, after 1 month) | unstable | unstable | unstable |
| | Usability (stickiness) | 3 | 2 | 2 |
| | Usability (flexibility) | 2 | 2 | 1 |

| | | | | |
|---|---|---|---|---|
| (% by mass) | | | | |

### <Experimental Example 3>

Aqueous solutions having a total surfactant concentration of 60% by mass were each prepared by mixing a first surfactant (polyglyceryl-5 oleate or PEG-12 diisostearate) and a second surfactant (Mal₂Far or arginine dodecylphosphate) in a range of 10:0 to 0:10 (mass ratio). For each of the thus obtained mixed aqueous solutions, the interfacial tension was measured by a spinning-drop interfacial tension measurement method (apparatus: SITE100MK2, manufactured by KRUSS GmbH; measurement temperature: 20°C) in terms of relative value with respect to polyperfluoromethylisopropyl ether (FOMBLIN HC/04, manufactured by Nikko Chemicals Co., Ltd.). In addition, the storage modulus G' was measured by a viscoelasticity measurement method using a rheometer (apparatus: AR-G2, manufactured by TA Instruments Japan Inc.; measurement mode: stress sweep, measurement temperature: 20°C, vibrational stress: 1 to 600 Pa, geometry: 40-mm cone, GAP value: 1,000).

Further, using each of the mixed aqueous solution as an intermediate phase, oil-in-water type emulsion compositions were prepared in accordance with the production method of the present invention. That is, to an aqueous solution having a total surfactant concentration of 60% by mass in an amount such that the final concentration of the total surfactant in whole composition was 1% by mass, glyceryl tri(caprylate/caprate) was added in an amount of 20% by mass with respect to the whole composition with heating and stirring, and the resulting mixture was homogenized, after which water was further added thereto in a residual amount, and the resultant was subsequently homogenized and cooled to 30°C with stirring. For the thus obtained oil-in-water type emulsion compositions, the size of emulsified particles and the temporal and temperature stability were evaluated in accordance with the above-described Test Examples 1 and 2, respectively.

The results thereof are shown in Tables 12 to 14.

### [Table 12]

**Table 12**

| | | | | | | |
|---|---|---|---|---|---|---|
| polyglyceryl-5 oleate:Mal₂Far^{∗1} (mass ratio) | 10:0 | 8:2 | 6:4 | 4:6 | 2:8 | 0:10 |
| Interfacial tension (mN/m)^{∗2} | 8.5 | 4.0 | 3.5 | 3.0 | 6.0 | 7.0 |
| G' | 1,397 | 11 | 12 | 69 | 146 | 17,390 |
| Evaluation of emulsified particles | ∘ | ∘ | ⊚ | ∘ | Δ | × |
| Temporal stability (at 40°C, 1 month) | Δ | ∘ | ∘ | ∘ | × | × |

| | | | | | | |
|---|---|---|---|---|---|---|
| ^{∗}1: glycoside of maltose and hexahydrofarnesol (1-O-3,7,11-trimethyldodecyl)-*β*-D-maltoside) ^{∗}2: relative value with respect to polyperfluoromethylisopropyl ether | | | | | | |

### [Table 13]

**Table 13**

| | | | | | | |
|---|---|---|---|---|---|---|
| polyglyceryl-5 oleate:arginine dodecylphosphate (mass ratio) | 10:0 | 8:2 | 6:4 | 4:6 | 2:8 | 0:10 |
| Interfacial tension (mN/m)^{∗} | 8.5 | 8.0 | 5.8 | 6.0 | 8.6 | 10.5 |
| G' | 1,397 | 21 | 16 | 33 | 619 | 18,740 |
| Evaluation of emulsified particles | ∘ | ⊚ | ⊚ | ∘ | Δ | Δ |
| Temporal stability (at 40°C, 1 month) | Δ | ∘ | ∘ | ∘ | Δ | × |

| | | | | | | |
|---|---|---|---|---|---|---|
| *relative value with respect to polyperfluoromethylisopropyl ether | | | | | | |

### [Table 14]

**Table 14**

| | | | | | | |
|---|---|---|---|---|---|---|
| PEG-12 diisostearate:arginine dodecylphosphate (mass ratio) | 10:0 | 8:2 | 6:4 | 4:6 | 2:8 | 0:10 |
| Interfacial tension (mN/m)^{∗} | 10.2 | 9.8 | 9.2 | 8.0 | 8.4 | 10.5 |
| G' | 2,620 | 1,240 | 640 | 240 | 11,320 | 18,740 |
| Evaluation of emulsified particles | × | Δ | Δ | ∘ | ∘ | Δ |
| Temporal stability (at 40°C, 1 month) | × | × | × | ∘ | Δ | × |

| | | | | | | |
|---|---|---|---|---|---|---|
| ^{∗}relative value with respect to polyperfluoromethylisopropyl ether | | | | | | |

### <Experimental Example 4> (not prepared according to the claimed method)

Oil-in-water type emulsion compositions were each prepared by a conventional method in accordance with the formulations shown in Table 15.

### [Table 15]

**Table 15 (% by mass)**

| | Reference Example 1 | Example 53 |
|---|---|---|
| polyglyceryl-5 oleate | 1 | 0.6 |
| Mal₂Far | 0 | 0.4 |
| glycerol | 10 | 10 |
| xanthan gum | 0.5 | 0.5 |
| squalane | 10 | 10 |
| citric acid | 0.01 | 0.01 |
| sodium citrate | 0.24 | 0.24 |
| water | 78.25 | 78.25 |

A cultured human epidermis model EpiDerm (registered trademark, manufactured by MatTek Corporation) was prepared, the oil-in-water type emulsion compositions of Reference Example 1 and Example 53 prepared above were each applied thereto, and a cross-section of the horny layer and corneocytes were observed 24 hours thereafter. The observation was performed under a light microscope using gentian violet and brilliant green as staining solutions. The thus obtained optical micrographs are shown in FIGs. 1 to 3 along with untreated cultured human epidermis model.

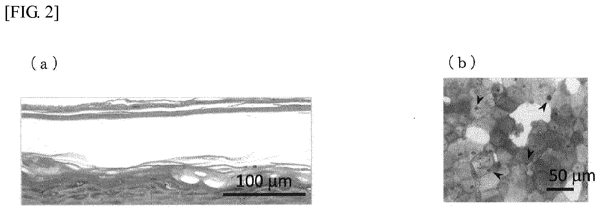

In the untreated cultured human epidermis model shown in FIG. 1(a), the horny layer is in a thin state and, in the cultured human epidermis model shown in FIG. 2(a) to which the oil-in-water type emulsion composition of Reference Example 1 was applied, peeling of the horny layer occurred. Meanwhile, in the cultured human epidermis model shown in FIG. 3(a) to which the oil-in-water type emulsion composition of Example 53 was applied, advanced lamination of horny layers can be seen.

Further, in the untreated cultured human epidermis model shown in FIG. 1(b) and the cultured human epidermis model shown in FIG. 2(b) to which the oil-in-water type emulsion composition of Reference Example 1 was applied, it was confirmed that nuclei remained in the horny layer cells. On the other hand, in the cultured human epidermis model shown in FIG. 3(b) to which the oil-in-water type emulsion composition of Example 53 was applied, enucleation can be seen.

### <Experimental Example 5> (not prepared according to the claimed method)

Oil-in-water type emulsion compositions were each prepared by a conventional method in accordance with the formulations shown in Table 16.

### [Table 16]

**Table 16 (% by mass)**

| | Reference Example 2 | Example 54 |
|---|---|---|
| polyglyceryl-5 oleate | 0 | 0.6 |
| Mal₂Far | 0 | 0.4 |
| methylglucose distearate polyglyceride | 1 | 0 |
| behenyl alcohol | 0.5 | 0 |
| fatty acid^{∗3} | 0.5 | 0 |
| oil component^{∗1} | 8.5 | 20 |
| polyhydric alcohol^{∗2} | 12 | 6.5 |
| pH-adjusting agent, preservative, and thickening agent | 1 or less | 1 or less |
| water | total = 100 | total = 100 |

| | | |
|---|---|---|
| ^{∗}1: The oil component comprises ethyl isostearate, capric acid triglyceride, squalane, sweet almond oil, jojoba oil, olive oil, shea tree, sunflower oil, and glyceryl oleate. ^{∗}2: The polyhydric alcohol comprises glycerol, diglycerol, and propanediol. ^{∗}3: The fatty acid comprises lauric acid, myristic acid, palmitic acid, stearic acid, oleic acid, and isostearic acid. | | |

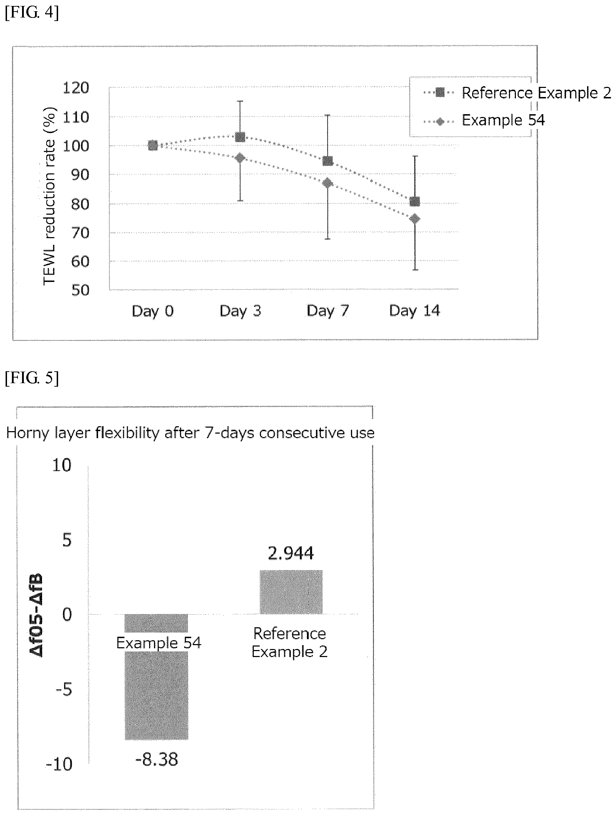

Twenty-four female panelists were asked to use the thus prepared oil-in-water type emulsion composition of Example 54 or Reference Example 2 for 14 days in a row, and the TEWL value of each panelist was measured on Day 3, Day 7 and Day 14. The measurement results are shown in FIG. 4.

The use of the oil-in-water type emulsion composition of Example 54 resulted in a significant reduction in the TEWL value by Day 7 after the start of the use, as compared to the use of the oil-in-water type emulsion composition of Reference Example 2.

Next, twelve panelists in their 20s to 50s were asked to use the oil-in-water type emulsion composition of Example 54 or Reference Example 2 that was prepared above for 7 days in a row, and the horny layer flexibility was measured using VENUSTRON III (manufactured by AXIOM) after 7 days since the start of the use.

In the measurement, first, in the initial stage before the use of the oil-in-water type emulsion composition, the change in frequency was measured as ΔfB at a probe pressure of 0.5 g, and the average thereof was calculated. Next, for those panelists after the 7-day use period, the change in frequency was measured as Δf0.5 at a probe pressure of 0.5 g, and the average thereof was calculated. Then, the value of (Δf0.5-ΔfB) was calculated. The measurement results are shown in FIG. 5.

The oil-in-water type emulsion composition of Example 54 yielded a negative (Δf0.5 - ΔfB) value; therefore, it is understood that the horny layer flexibility was improved as compared to before the use.

### INDUSTRIAL APPLICABILITY

According to the present invention, an oil-in-water emulsion composition which has excellent temporal and temperature stability and exerts a good feeling of use can be produced. This composition is industrially very useful because it can be suitably used for skin external preparations and the like and is particularly suitable as a cosmetic.

## Claims

1. A method of producing an oil-in-water type emulsion composition, said method comprising:
the step of forming an intermediate phase by mixing a first surfactant, a second surfactant, and water;
the step of adding an oil phase component to said intermediate phase; and
the step of further adding an aqueous phase component,
wherein
said first surfactant forms an L_{α} phase in at least one aqueous solution having a concentration of 40 to 80% by mass, and
said second surfactant forms an H₁ phase in at least one aqueous solution having a concentration of 40 to 80% by mass,
wherein said oil-in-water type emulsion composition is preferably a skin external preparation, more preferably a cosmetic, still more preferably a skin external preparation for use in maturation of skin horny layer,
wherein the total concentration of said first and second surfactants in said intermediate phase is 20 to 85% by mass.

2. The method according to claim 1, wherein said intermediate phase has an interfacial tension of 0.01 to 8 mN/m in terms of relative value with respect to
polyperfluoromethylisopropyl ether.

3. The method according to claim 1 or 2, wherein a combination of said first surfactant and said second surfactant is a combination in which the viscosity of a mixed aqueous solution of said first and second surfactants is less than half of the sum of the viscosity values of individual aqueous solutions of said first and second surfactants at the same concentration as the total concentration of said surfactants in said mixed aqueous solution,
preferably, wherein said first surfactant comprises at least one selected from surfactin and salts thereof, mannosylerythritol lipids, polyglycerol fatty acid esters, sophorolipids, polyoxyethylene glyceryl fatty acids, polyethylene glycol difatty acids, and phospholipids,
preferably wherein said second surfactant comprises at least one selected from alkyl glycosides represented by the following Formula (1), acylamino acid lysine and/or salts thereof, sucrose fatty acid esters, polyglycerol alkyl ethers, fatty acid glycosides, and monoalkyl phosphates and salts thereof: 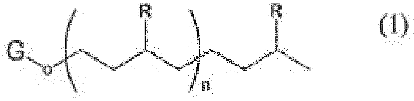
(wherein, G represents a sugar residue; Rs independently represent a hydrogen atom or an alkyl group having 1 to 3 carbon atoms; and n represents an integer of 1 to 4), and
more preferably wherein said first surfactant and/or said second surfactant comprise no ethylene oxide (EO) group.

4. The method according to any one of claims 1 to 3, wherein the concentration of said first surfactant is 0.1 to 5% by mass with respect to the whole composition.

5. The method according to any one of claims 1 to 4, wherein the concentration of said second surfactant is 0.1 to 5% by mass with respect to the whole composition.

6. The method according to any one of claims 1 to 5, wherein the total concentration of said first and second surfactants is 0.2 to 8% by mass with respect to the whole composition.

7. The method according to any one of claims 1 to 6, wherein the mass ratio of said first surfactant and said second surfactant is 2:8 to 8:2.

8. The method according to any one of claims 1 to 7, wherein the content of said oil phase component is 3 to 75% by mass with respect to the whole composition.

9. The method according to any one of claims 1 to 8, which comprises the step of incorporating a polyhydric alcohol in an amount of 1 to 30% by mass with respect to the whole composition.

## Patentansprüche

1. Verfahren zur Herstellung einer Emulsionszusammensetzung vom Öl-in-Wasser-Typ, wobei das Verfahren umfasst:
den Schritt der Bildung einer Zwischenphase durch Mischen eines ersten Tensids, eines zweiten Tensids und Wasser;
den Schritt der Zugabe einer Ölphasenkomponente zu der Zwischenphase; und
den Schritt der weiteren Zugabe einer wässrigen Phasenkomponente, wobei
das erste Tensid eine L_{α},-Phase in mindestens einer wässrigen Lösung mit einer Konzentration von 40 bis 80 Gew.-% bildet, und
das zweite Tensid eine H₁-Phase in mindestens einer wässrigen Lösung mit einer Konzentration von 40 bis 80 Gew.-% bildet,
wobei die Öl-in-Wasser-Emulsionszusammensetzung vorzugsweise ein äußerliches Hautpräparat, bevorzugter ein Kosmetikum, noch bevorzugter ein äußerliches Hautpräparat zur Verwendung bei der Reifung der Hornschicht der Haut ist,
wobei die Gesamtkonzentration der ersten und zweiten Tenside in der Zwischenphase 20 bis 85 Gew.-% beträgt.

2. Verfahren nach Anspruch 1, wobei die Zwischenphase eine Grenzflächenspannung von 0,01 bis 8 mN/m als relativen Wert in Bezug auf Polyperfluormethylisopropylether aufweist.

3. Verfahren nach Anspruch 1 oder 2, wobei eine Kombination des ersten Tensids und des zweiten Tensids eine Kombination ist, bei der die Viskosität einer gemischten wässrigen Lösung des ersten und des zweiten Tensids weniger als die Hälfte der Summe der Viskositätswerte der einzelnen wässrigen Lösungen des ersten und des zweiten Tensids bei derselben Konzentration wie die Gesamtkonzentration der Tenside in der gemischten wässrigen Lösung beträgt,
wobei das erste Tensid vorzugsweise mindestens eine Komponente umfasst, die ausgewählt ist aus Surfactin und dessen Salzen, Mannosylerythritol-Lipiden, Polyglycerinfettsäureestern, Sophorolipiden, Polyoxyethylenglycerylfettsäuren, Polyethylenglycoldifettsäuren und Phospholipiden,
wobei das zweite Tensid vorzugsweise mindestens eine Komponente umfasst, die ausgewählt ist aus Alkylglycosiden der folgenden Formel (1), Acylaminosäure-Lysin und/oder Salzen davon, Saccharosefettsäureestern, Polyglycerinalkylether, Fettsäureglycosiden und Monoalkylphosphaten und Salzen davon: 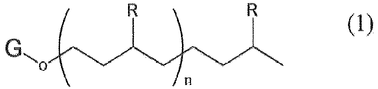
(wobei G einen Zuckerrest darstellt; die Rs unabhängig ein Wasserstoffatom oder eine Alkylgruppe mit 1 bis 3 Kohlenstoffatomen darstellen; und n eine ganze Zahl von 1 bis 4 darstellt), und
wobei das erste Tensid und/oder das zweite Tensid bevorzugter keine Ethylenoxid (EO)-Gruppe enthalten.

4. Verfahren nach einem der Ansprüche 1 bis 3, wobei die Konzentration des ersten Tensids 0,1 bis 5 Gew.-%, bezogen auf die gesamte Zusammensetzung, beträgt.

5. Verfahren nach einem der Ansprüche 1 bis 4, wobei die Konzentration des zweiten Tensids 0,1 bis 5 Gew.-%, bezogen auf die gesamte Zusammensetzung, beträgt.

6. Verfahren nach einem der Ansprüche 1 bis 5, wobei die Gesamtkonzentration des ersten und zweiten Tensids 0,2 bis 8 Gew.-%, bezogen auf die gesamte Zusammensetzung, beträgt.

7. Verfahren nach einem der Ansprüche 1 bis 6, wobei das Massenverhältnis des ersten Tensids und des zweiten Tensids 2:8 bis 8:2 beträgt.

8. Verfahren nach einem der Ansprüche 1 bis 7, wobei der Gehalt der Ölphasenkomponente 3 bis 75 Gew.-%, bezogen auf die gesamte Zusammensetzung, beträgt.

9. Verfahren nach einem der Ansprüche 1 bis 8, das den Schritt der Einarbeitung eines mehrwertigen Alkohols in einer Menge von 1 bis 30 Gew.-%, bezogen auf die gesamte Zusammensetzung, umfasst.

## Revendications

1. Procédé de production d'une composition d'émulsion de type huile dans eau,
procédé comprenant :
- une étape de formation d'une phase intermédiaire en mélangeant un premier surfactant, un second surfactant et de l'eau,
- une étape d'addition d'un composant de phase huile à la phase intermédiaire, et
- une étape d'addition supplémentaire d'un composant de phase aqueuse,
procédé selon lequel
- le premier surfactant forme une phase L_{α} dans au moins une solution aqueuse ayant une concentration massique de 40-80%, et
- le second surfactant forme une phase H₁ dans au moins une solution aqueuse ayant une concentration massique de 40-80%,
dans lequel
la composition de l'émulsion de type huile dans eau est de préférence une préparation externe pour la peau et de manière plus préférentielle, un cosmétique, de manière encore plus préférentielle, une préparation externe pour la peau, utilisée dans la maturation de la couche de peau cornée,
- la concentration totale du premier et du second surfactant dans la phase intermédiaire est une concentration massique comprise entre 20 et 85%.

2. Procédé selon la revendication 1,
selon lequel
la phase intermédiaire a une tension interfaciale de 0,01-8 mN/m en termes de valeur relative par rapport au poly-fluoro-méthyl-isopropyle éther.

3. Procédé selon la revendication 1 ou 2,
selon lequel
une combinaison du premier surfactant et du second surfactant est une combinaison dans laquelle la viscosité de la solution aqueuse mélangée du premier et du second surfactant est inférieure à la moitié de la somme des viscosités des solutions aqueuses prises individuellement du premier et du second surfactant à la même concentration que la concentration totale des surfactants dans cette solution aqueuse mélangée,
- de préférence, le premier surfactant comprend au moins la surfactine et ses sels : lipides mannosylerythritole, esters d'acides gras de polyglycérol, sophorolipides, polyoxyéthylène glycéryl, acides gras, polyéthylène glycol, acides gras, et phospholipides,
- de préférence, dans lequel le deuxième tensio actif comprend au moins un des alkyl-glycosides, et phosphates monoalkyl et leurs sels : 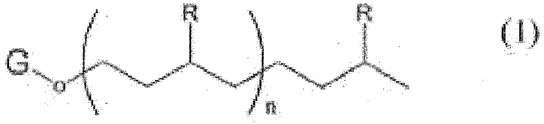 (formule dans lequel G représente un résidu de sucre ; Rs indépendamment représente un atome d'hydrogène ou un groupe alkyle ayant entre 1 et 3 atomes de carbone et (n) représente un nombre entier compris entre 1 et 4), et
- de façon plus préférentielle, le premier surfactant et/ou le second surfactant ne comportent pas de groupe éthylène-oxyde (EO).

4. Procédé selon l'une quelconque des revendications 1 à 3,
selon lequel
la concentration du premier surfactant est comprise dans un pourcentage massique entre 0,1 et 5% par rapport à la composition totale.

5. Procédé selon l'une quelconque des revendications 1 à 4,
selon lequel
la concentration du second surfactant est comprise dans un pourcentage massique entre 0,1 et 5% par rapport à la composition totale.

6. Procédé selon l'une quelconque des revendications 1 à 5,
selon lequel
la concentration totale du premier et du second surfactants est comprise dans un pourcentage massique entre 0,2 et 8% par rapport à la composition totale.

7. Procédé selon l'une quelconque des revendications 1 à 6,
selon lequel
le rapport massique du premier surfactant ou second surfactant est compris entre 2/8 à 8/2.

8. Procédé selon l'une quelconque des revendications 1 à 7,
selon lequel
le contenu du composant de phase huile est comprise dans un pourcentage massique de 3 à 75% par rapport à la composition totale.

9. Procédé selon l'une quelconque des revendications 1 à 8,
selon lequel
il comprend l'étape d'incorporation de l'alcool polyhydrique selon un pourcentage massique compris entre 1 et 30% par rapport à la composition totale.
